# EUROPEAN PATENT SPECIFICATION

(11) **EP 4 331 632 B1**
(45) Date of publication and mention of the grant of the patent: **14.10.2026**
(21) Application number: 23209506.7
(22) Date of filing: 29.04.2016
(51) Int. Cl.: A61L 27/14, A61L 27/38, D04H 1/728, D04H 1/435, D04H 1/4266, A61L 27/60, A61L 27/58, A61L 27/48, A61L 15/64, A61L 15/40

(54) **MULTI-COMPONENT ELECTROSPUN FIBER SCAFFOLDS**
MEHRKOMPONENTIGE ELEKTROGESPONNENE FASERGERÜSTE
MATRICES EN FIBRES ÉLECTROFILÉES MULTI-COMPOSANT

(30) Priority: 29.04.2015 US 201562154286 P
(43) Date of publication of application: 06.03.2024
(62) Document number(s) of the earlier application(s) in accordance with Art. 76 EPC: 16787222.5 / 3 288 602
(73) Proprietor: NFS IP Holdings, LLC, Dublin, OH 43017 (US)
(72) Inventor: JOHNSON, Jed, Columbus, OH 43212 (US)
(74) Representative: HGF

(56) References cited:
- WO-A1-2015/048224
- AU-B2- 2009 207 489
- US-A1- 2012 093 717

## Description

### CROSS-REFERENCE TO RELATED APPLICATIONS

This application claims priority to and benefit of U.S. Provisional Application Serial No. 62/154,286 filed April 29, 2015, entitled "Multi-Component Nanofiber Scaffolds."

### BACKGROUND

Over $25 billion is spent annually on acute and chronic non-healing wounds, illustrating an unmet clinical need for effective, inexpensive wound healing innovations. In the diabetic population alone, 25% of patients will develop a foot ulcer that will end with an amputation. Cell-based wound dressings are prohibitively expensive, and therefore are often not a viable option for patients with non-healing wounds.

In addition to the need for wound healing innovations, there exists a need for artificial vascular grafts capable of effectively mimicking the mechanics of native blood vessels, while promoting cellular ingrowth. Approximately 30% of all deaths in the United States are the result of cardiovascular disease (CVD), and 1.4 million patients annually in the U.S. require arterial prostheses. Coronary and peripheral vascular bypass graft procedures are performed in approximately 600,000 patients annually in the U.S., most commonly with the saphenous vein or the internal mammary artery. Although the use of autologous vascular substitutes has had a major impact on advancing the field of reconstructive vascular surgery, these tissue sources may be inadequate or unavailable in up to 20% of patients due to comorbidities, and the replicability of grafts from older donors is limited. Moreover, their harvest adds time, cost, and the potential for additional morbidity to the surgical procedure.

According to the American Society of Nephrology, more than 300,000 Americans have end-stage renal disease (ESRD) and depend on artificial dialysis to survive. Arteriovenous (AV) fistulae are commonly constructed to create vascular access for hemodialysis. However, access failure is currently one of the leading causes of hospitalization for patients with ESRD. Infection and early thrombosis of synthetic grafts, such as those made from expanded polytetrafluoroethylene (ePTFE), and intimal hyperplasia of AV fistulas prevent these procedures from having better success rates. Currently, ePTFE, polyethylene terephthalate (PET), and polyurethane (PU) are used to fabricate synthetic vascular grafts. However, neointimal hyperplasia formation due to a lack of endothelialization, infection, thrombosis, and compliance mismatch to the native vasculature are problems with these synthetics, and none of these materials have proven suitable for generating successful grafts less than 6 mm in diameter.

Matching native vessel compliance is a critical attribute of a vascular graft, and requires the ability to finely tune the mechanical properties of the graft to that of the native vasculature. Compliance mismatch between the graft and the native vessel disrupts blood flow, resulting in zones of recirculation, flow separation, and low wall shear stress. Low wall shear stress initiates the release of vasoactive substances, gene activation, protein expression, and cytoskeletal rearrangement that stimulate neointimal hyperplasia. This is seen as a cause of failure in both autologous and prosthetic bypass grafts, though saphenous vein grafts also suffer from stenosis at sites away from the anastomosis.

Polymer fibers may mimic the physical structure found within the body and promote cellular remodeling and wound healing. Scaffolds comprised of such fibers may be effective in both wound healing and vascular grafting applications. US 2012/093717 discloses composites for drug delivery comprising a combination of polymeric fibers having different degradation rates.

### SUMMARY

In a first aspect of the invention, there is provided an electrospun polymer sheet comprising:
a plurality of fibers co-electrospun from a solution comprising a first polymer and a second polymer;
wherein the first polymer and the second polymer comprise at least one of polylactic acid, polyglycolide, poly(lactide-co-caprolactone), or poly(lactide-co-glycolide); wherein the first polymer comprises a first degradation rate when exposed to a bodily tissue or a fluid and the second polymer comprises a second degradation rate when exposed to the bodily tissue or the fluid, wherein the second degradation rate differs from the first degradation rate;
wherein degradation of the first polymer causes the electrospun polymer sheet to form a scaffold configured to support cellular ingrowth into the scaffold.

In second aspect of the invention, there is provided a method of making an electrospun polymer sheet, the method comprising:
preparing a first solution comprising a first polymer;
preparing a second solution comprising a second polymer, wherein the first polymer and the second polymer comprise at least one of polylactic acid, polyglycolide, poly(lactide-cocaprolactone), or poly(lactide-co-glycolide), wherein the first polymer comprises a first degradation rate when exposed to a bodily tissue or a fluid and the second polymer comprises a second degradation rate when exposed to the bodily tissue or the fluid, wherein the second degradation rate differs from the first degradation rate;
applying a charge to one or more of a receiving surface, a polymer injection system, and a solution ejected from the polymer injection system; and
simultaneously ejecting, via the polymer injection system, the first solution and the second solution onto the receiving surface to form the electrospun polymer sheet comprising a plurality of fibers, wherein the electrospun polymer sheet is configured such that degradation of the first polymer causes the electrospun polymer sheet to form a scaffold configured to support cellular ingrowth into the scaffold.

In an embodiment, a scaffold may comprise a first polymeric electrospun fiber comprising a first material that has a first degradation rate, and a second polymeric electrospun fiber comprising a second material that has a second degradation rate different from the first degradation rate. In some embodiments, the first degradation rate may be higher or faster than the second degradation rate.

In another embodiment, a scaffold may comprise a first polymeric electrospun fiber comprising a first material having a first degradation rate that substantially corresponds to a cell infiltration rate, and a second polymeric electrospun fiber comprising a second material having a second degradation rate slower than the first degradation rate.

Described herein is a kit comprising a scaffold having a first polymeric electrospun fiber comprising a first material that has a first degradation rate, and a second polymeric electrospun fiber comprising a second material that has a second degradation rate different from the first degradation rate, and a sealable pouch.

In an embodiment of the second aspect of the invention, the method may comprise electrospinning a first polymer fiber having a first degradation rate by ejecting a first polymer solution from a first polymer injection system onto a mandrel, and electrospinning a second polymer fiber having a second degradation rate different from the first degradation rate by ejecting a second polymer solution from a second polymer injection system onto the mandrel. In some embodiments, the electrospinning steps may be performed simultaneously.

Described herein is a method of improving wound healing comprising applying to a portion of a wound a scaffold comprising a first polymeric electrospun fiber comprising a first material having a first degradation rate, and a second polymeric electrospun fiber having a second degradation rate different from the first degradation rate.

### BRIEF DESCRIPTION OF THE DRAWINGS

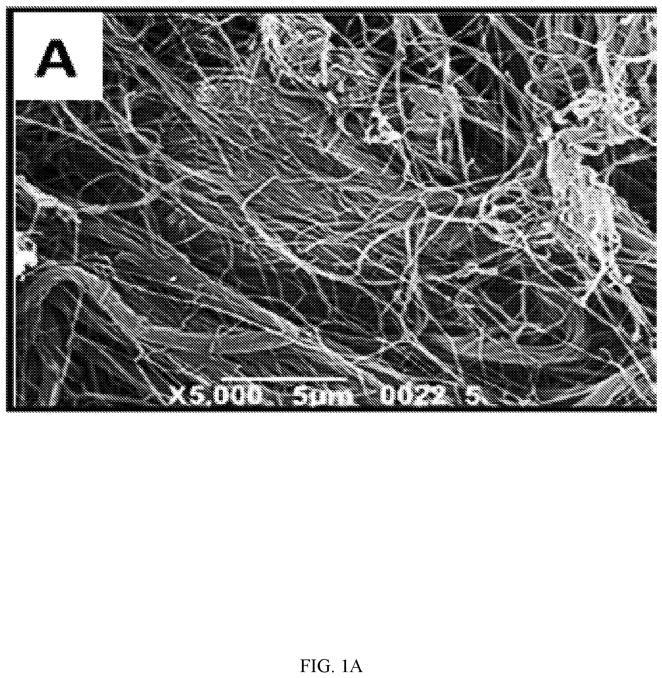

FIG. 1A illustrates a scanning electron microscope image of decellularized tissue.

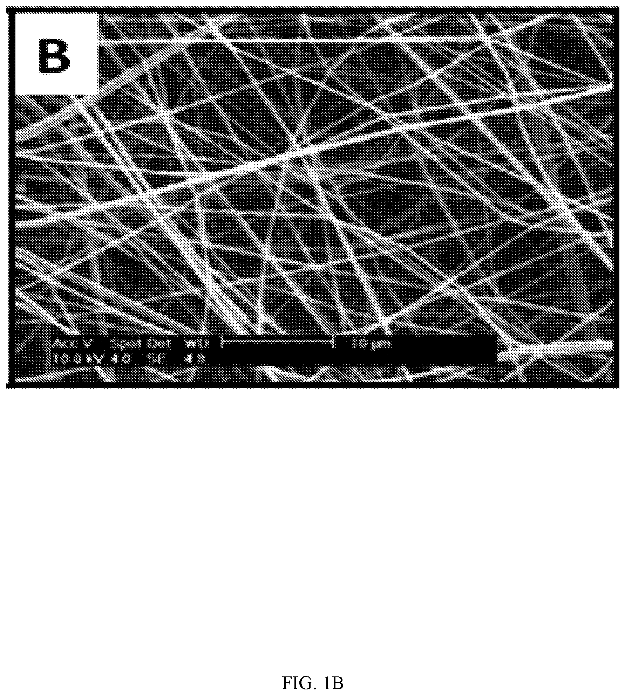

FIG. 1B illustrates a scanning electron microscope image of an embodiment of a scaffold in accordance with the present disclosure. The scaffold of FIG. 1B shares many microstructural similarities with the decellularized tissue of FIG. 1A.
FIGs. 2A-2F illustrate the healing of a wound with no scaffold.

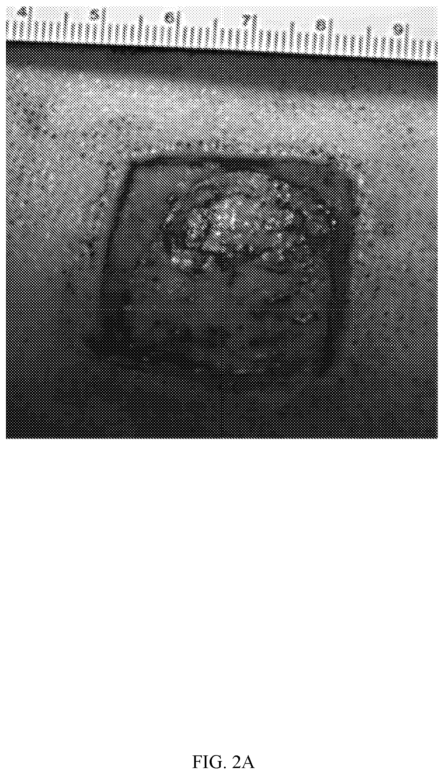

FIG. 2A illustrates the wound at day 0.

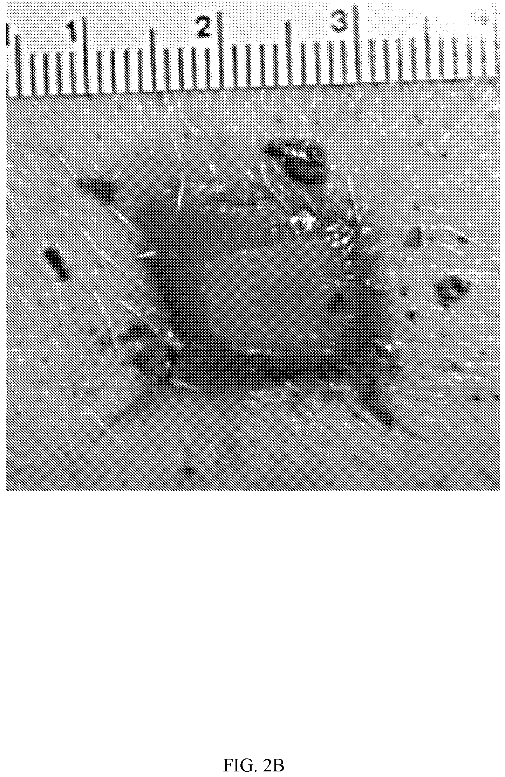

FIG. 2B illustrates the same wound at day 7, with 33% wound closure.

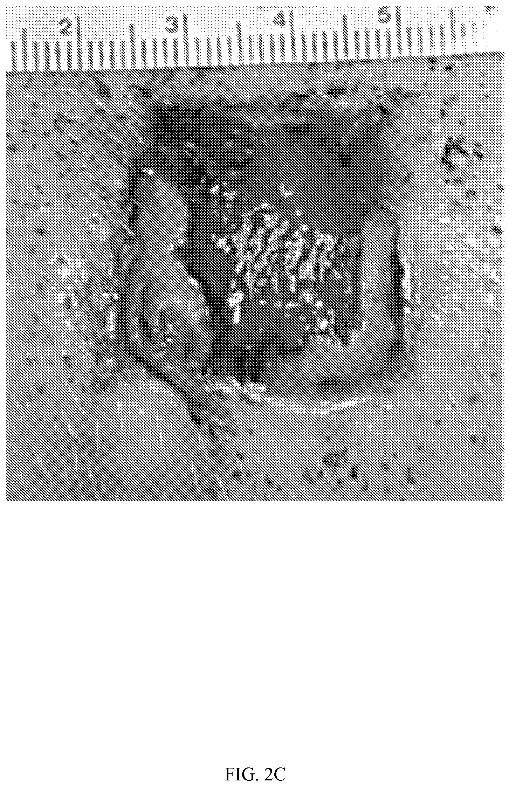

FIG. 2C illustrates the same wound at day 14, with 78% wound closure.

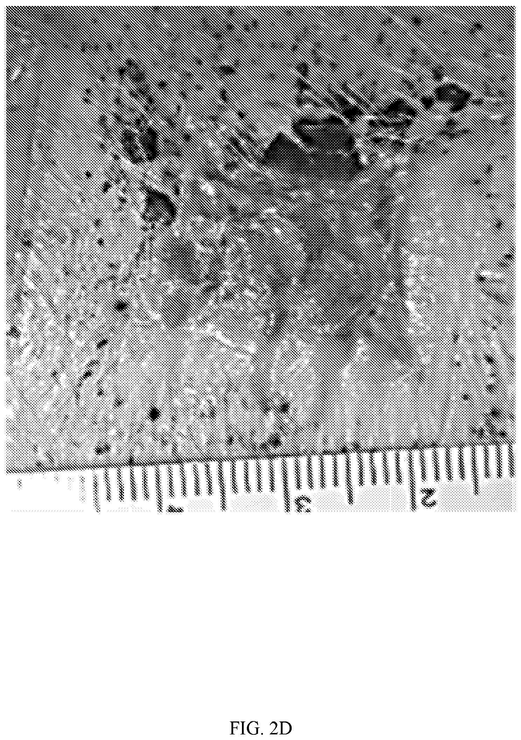

FIG. 2D illustrates the same wound at day 21, with 97% wound closure.

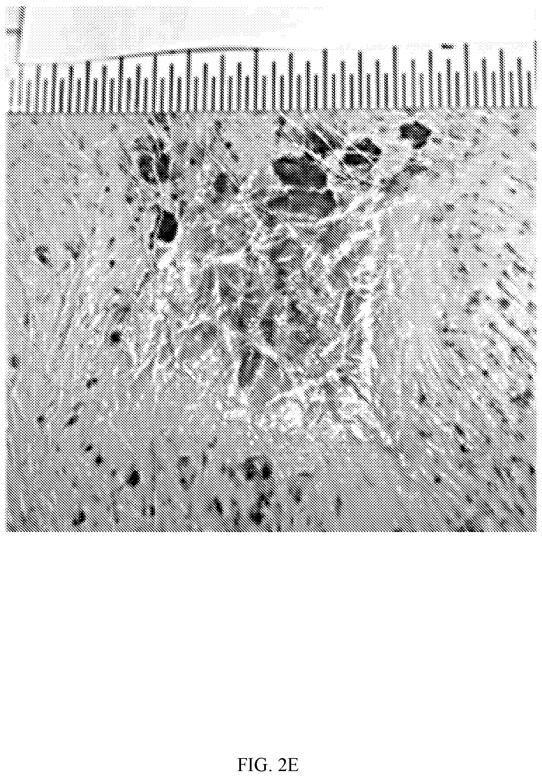

FIG. 2E illustrates the same wound at day 28, with 100% wound closure.

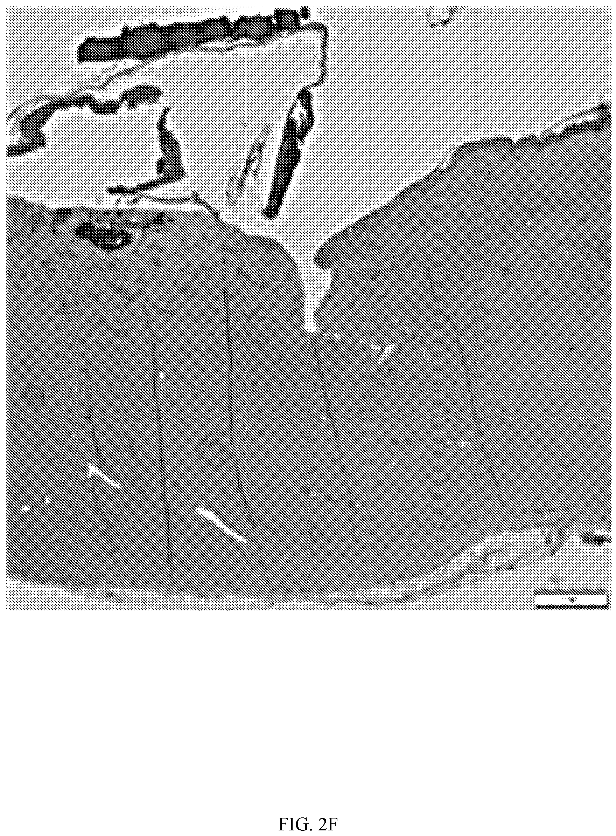

FIG. 2F illustrates histology of the wound at day 28. The histology showed an incomplete epidermal layer with a gap of approximately 2/3 of the wound. The scab over the wound had abundant neutrophils, and fibrous tissue under the wound was about twice as thick as the adjacent fibrous layers. Mild to moderate diffuse subcutaneous lymphocyte infiltration was present.

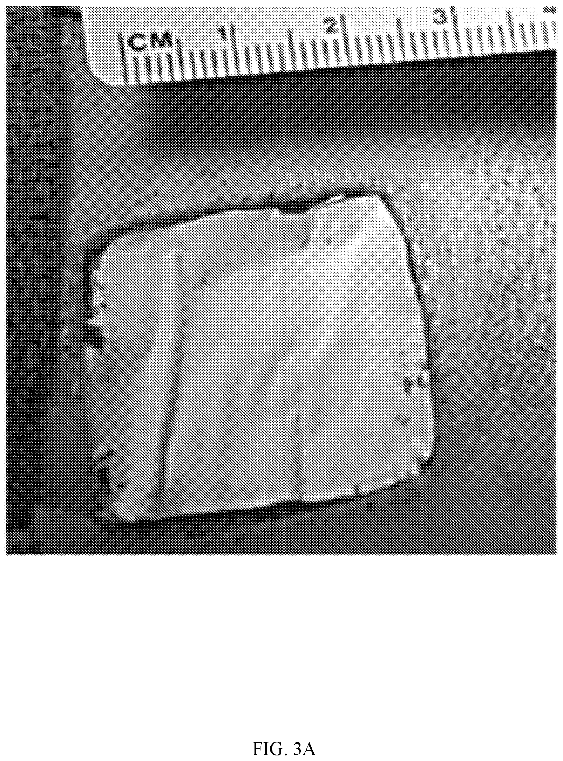

FIGs. 3A-3F illustrate the healing of a wound with an embodiment of a scaffold applied at day 0, in accordance with the present disclosure.
FIG. 3A illustrates the wound with the scaffold at day 0.

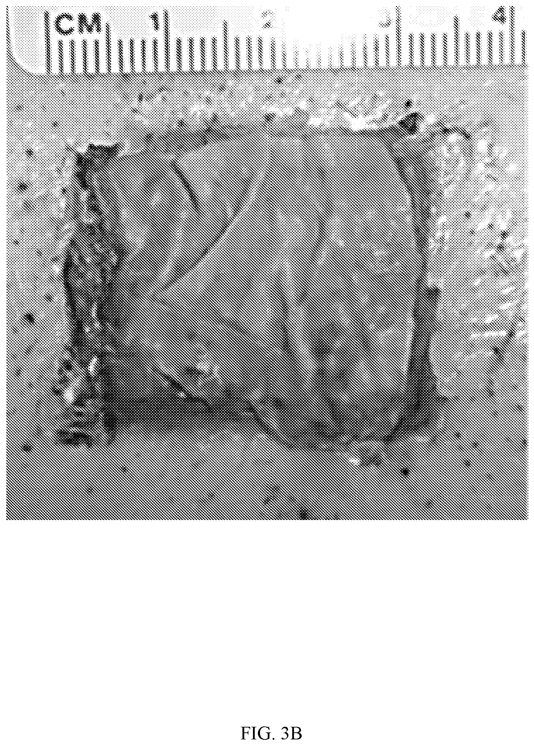

FIG. 3B illustrates the same wound with the same scaffold at day 7, with 28% wound closure.

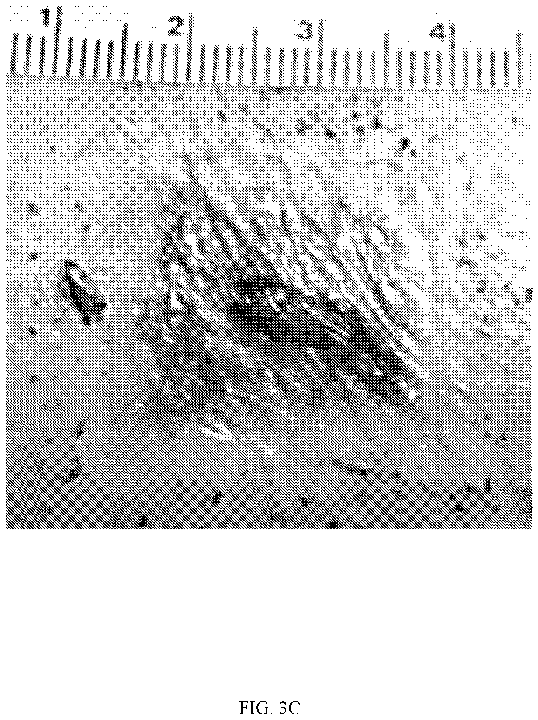

FIG. 3C illustrates the same wound with the same scaffold at day 14, with 96% wound closure.

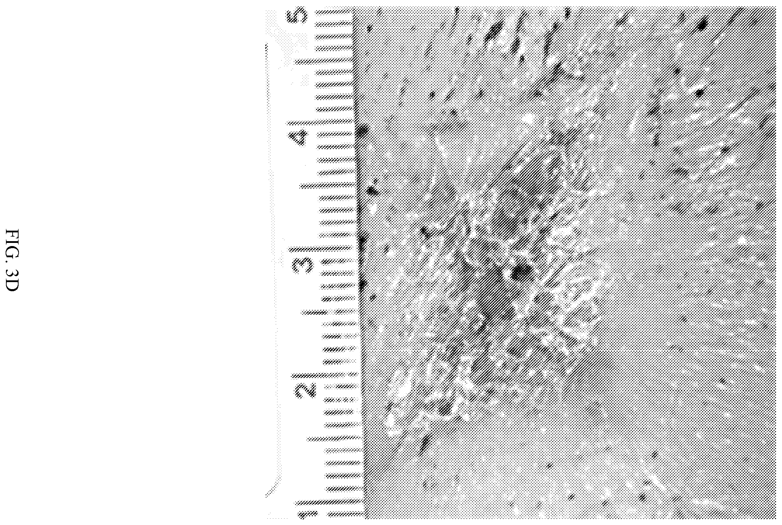

FIG. 3D illustrates the same wound with the same scaffold at day 21, with 100% wound closure.

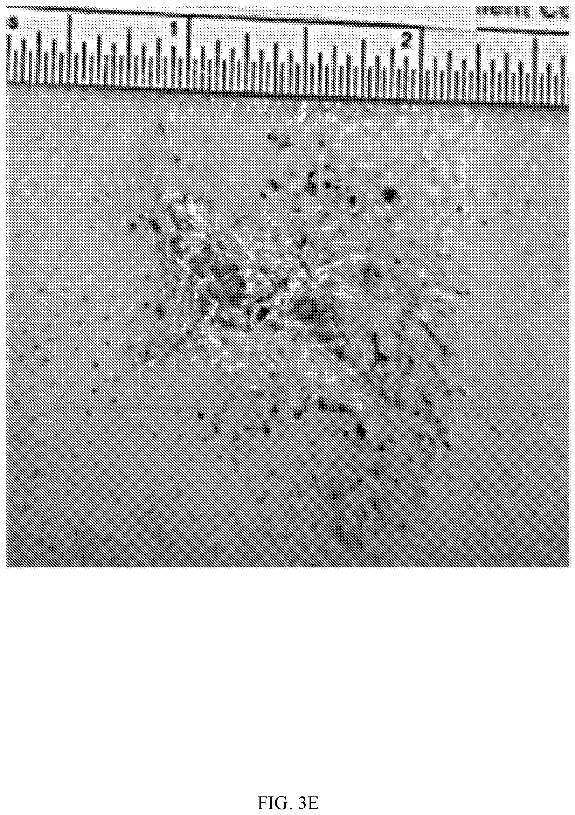

FIG. 3E illustrates the same wound with the same scaffold at day 28, with 100% wound closure.

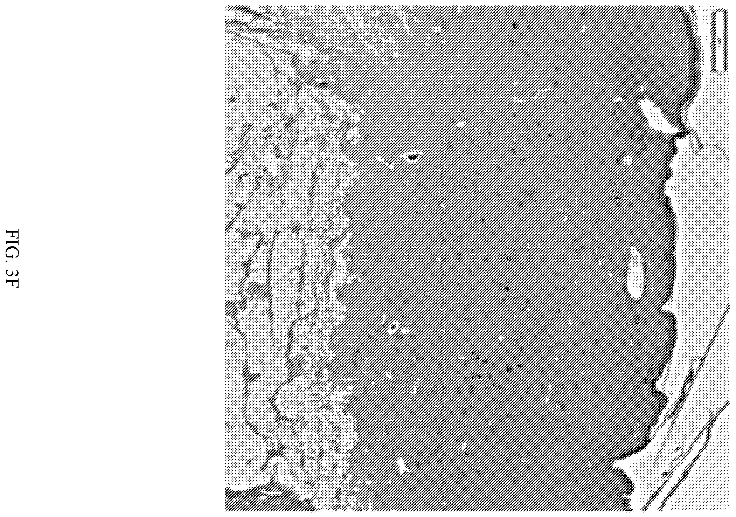

FIG. 3F illustrates histology of the wound with the scaffold at day 28. The histology showed a complete epidermal layer over the wound site with normal thickness and keratin production. Fibrous tissue under the wound was about twice as thick as the adjacent fibrous layers. Mild to moderate diffuse subcutaneous lymphocyte infiltration was present.

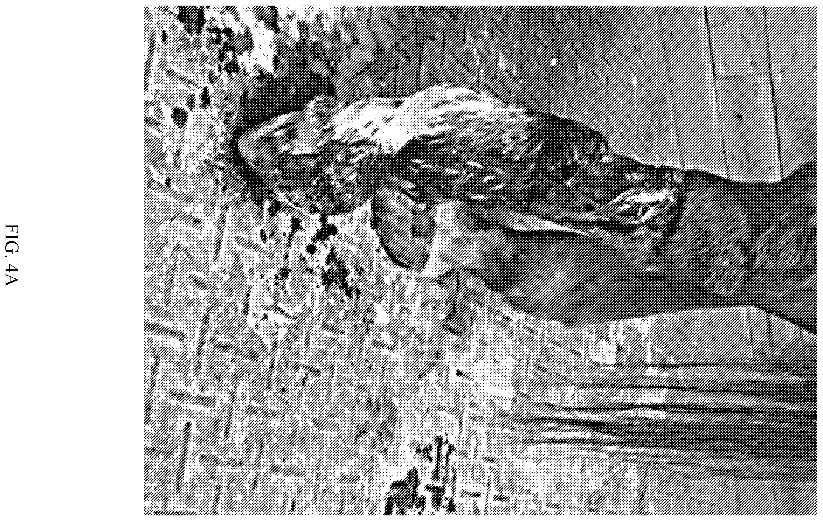

FIGs. 4A-4F illustrate the healing process of a horse with a severe leg injury cause by a high-tensile wire.
FIG. 4A illustrates the initial high-tensile wire wounds on the pastern and cannon bone.

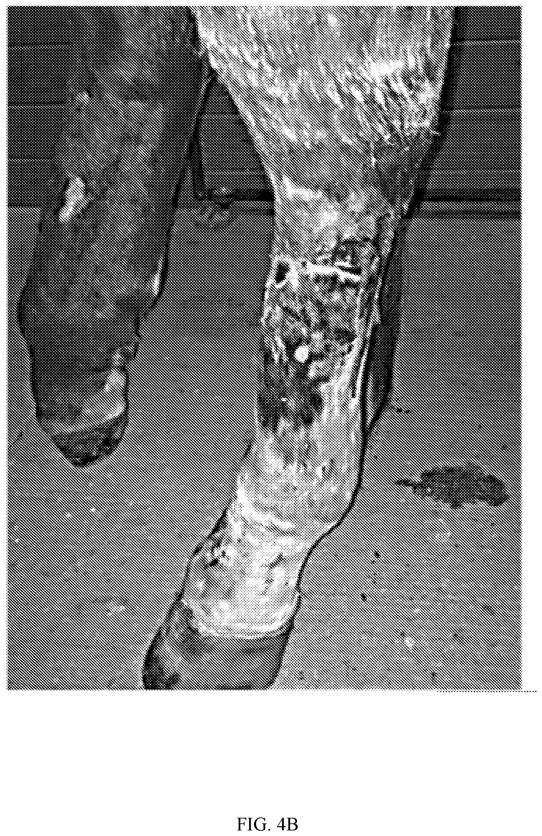

FIG. 4B illustrates both wounds fully healed 5 weeks after applying a scaffold in accordance with the present disclosure.

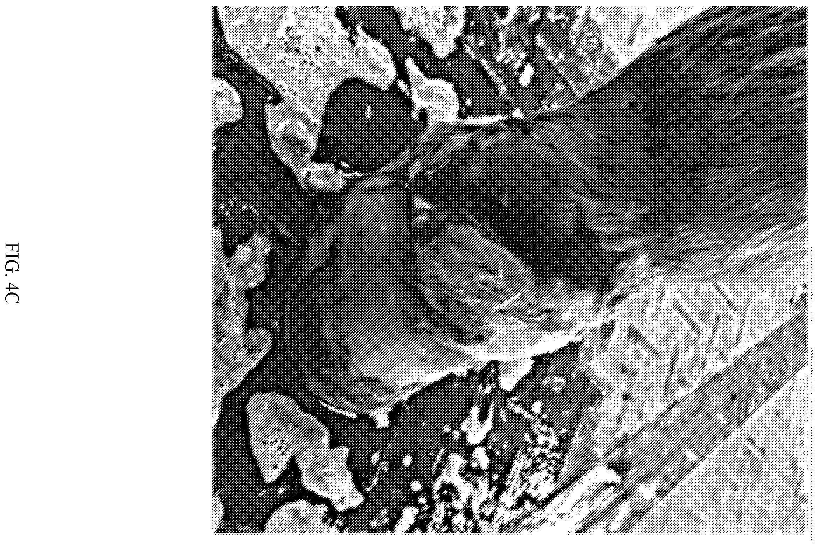

FIG. 4C illustrates the vascular wound to the pastern at the time of injury.

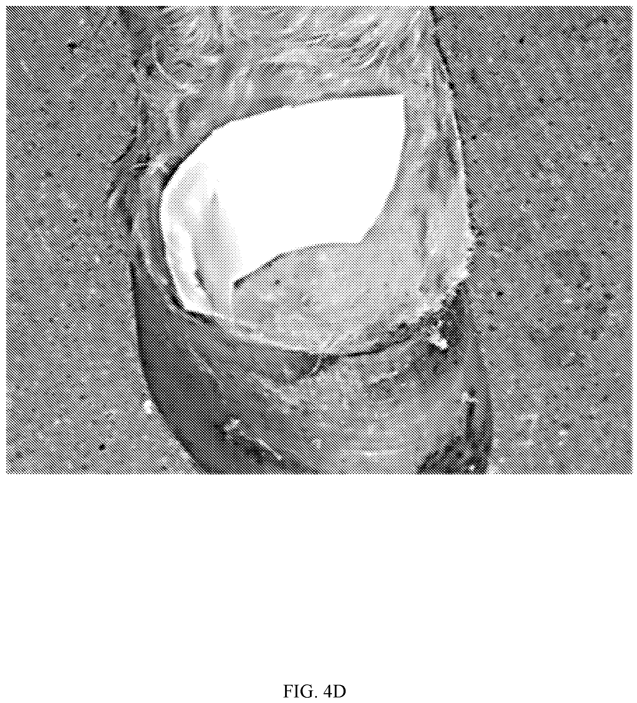

FIG. 4D illustrates the same vascular wound to the pastern, covered with a scaffold in accordance with the present disclosure.

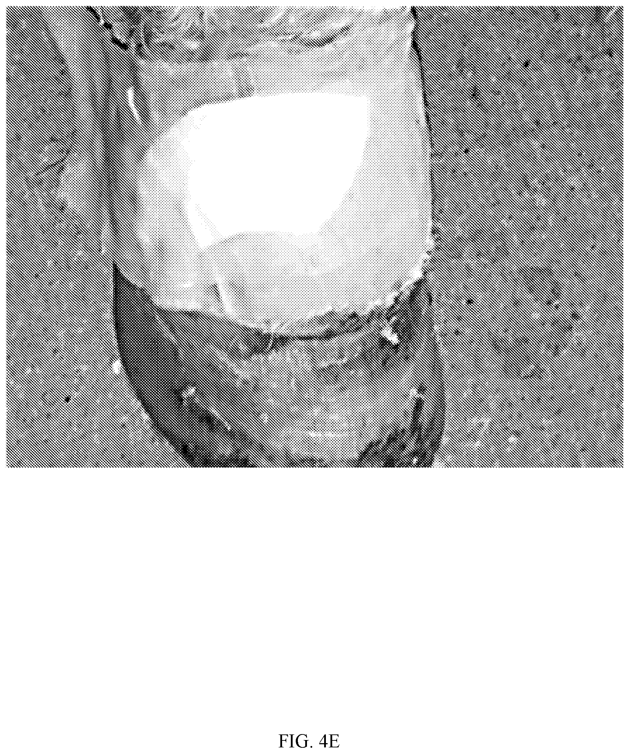

FIG. 4E illustrates a paraffin gauze bandage over the scaffold covering the pastern wound, in accordance with the present disclosure.

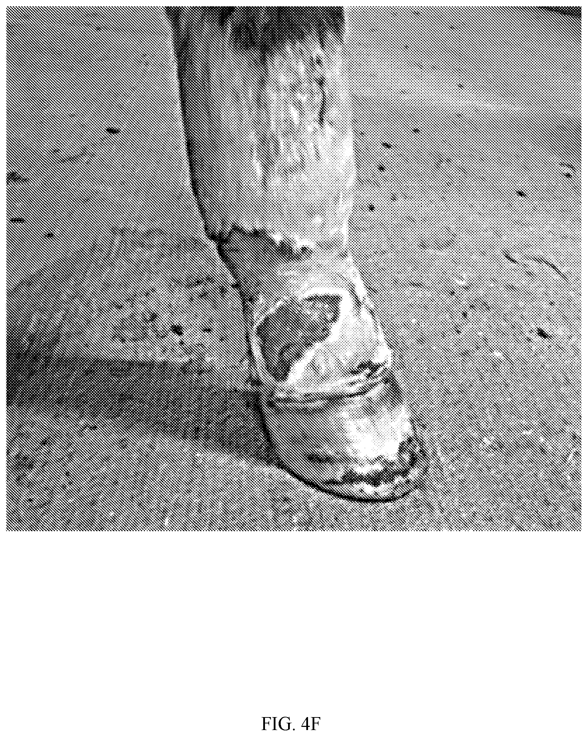

FIG. 4F illustrates the pastern wound healing 4 weeks after the scaffold was applied.

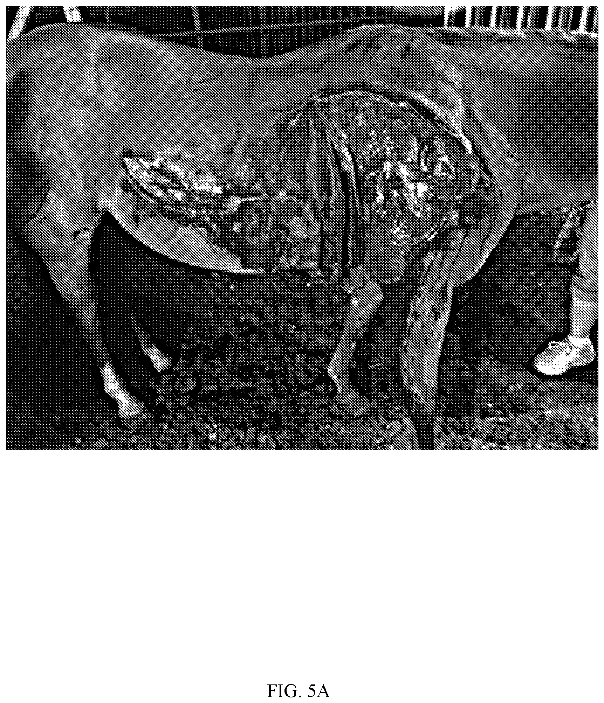

FIGs. 5A-5E illustrate the healing process of a horse with a large shoulder and torso laceration caused by running into a hay rake.
FIG. 5A illustrates the laceration at the time of injury.

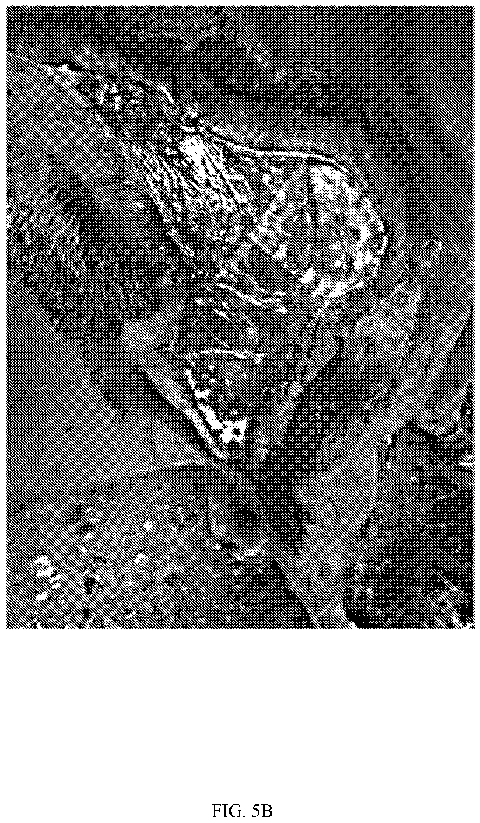

FIG. 5B illustrates the laceration at the time of injury, with a scaffold applied to the wound in accordance with the present disclosure.

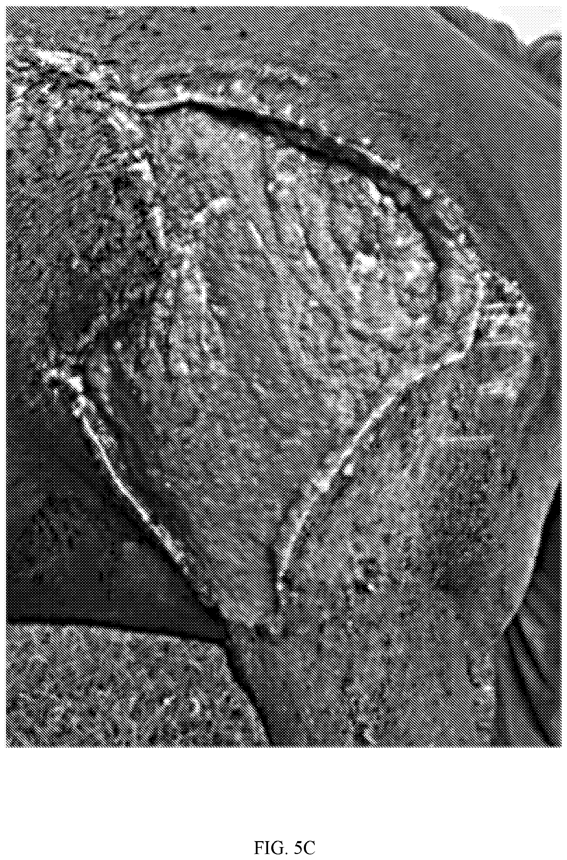

FIG. 5C illustrates the wound after 4 weeks of healing, with evidence of granulation tissue regrowth.

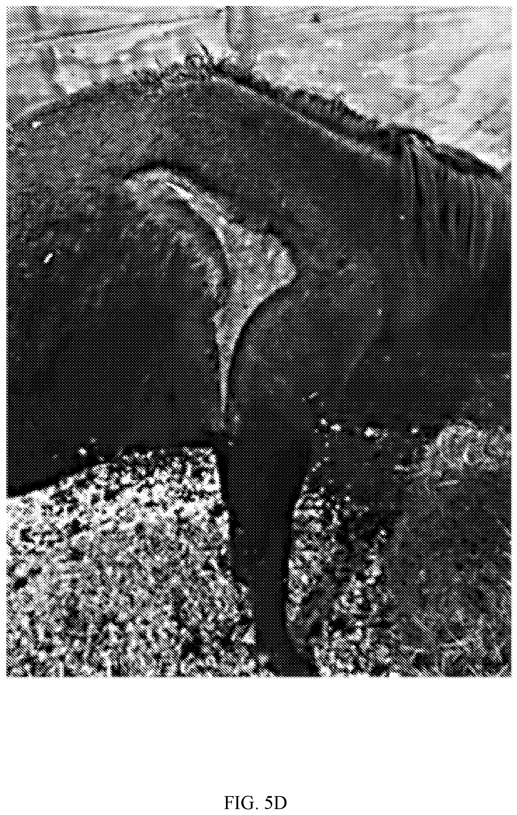

FIG. 5D illustrates the wound after 2 months (8 weeks) of healing, with evidence of epithelialization and hair regrowth.

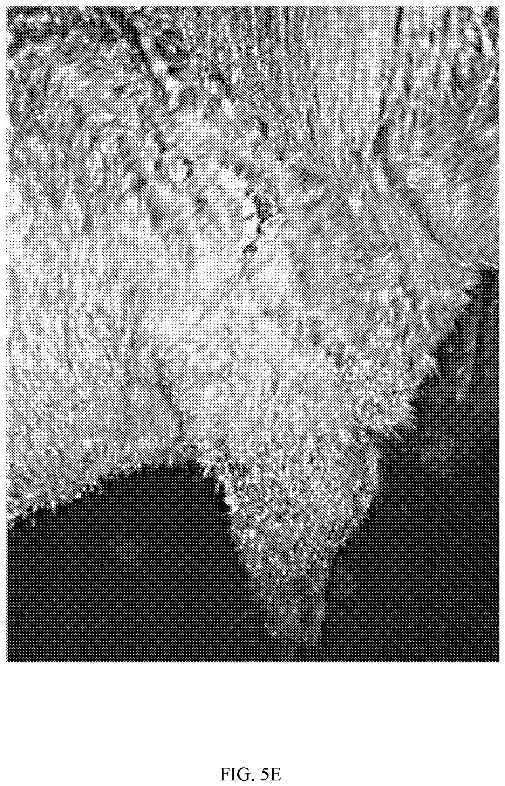

FIG. 5E illustrates the wound after 5 months (20 weeks) of healing, with further epithelialization and hair regrowth.

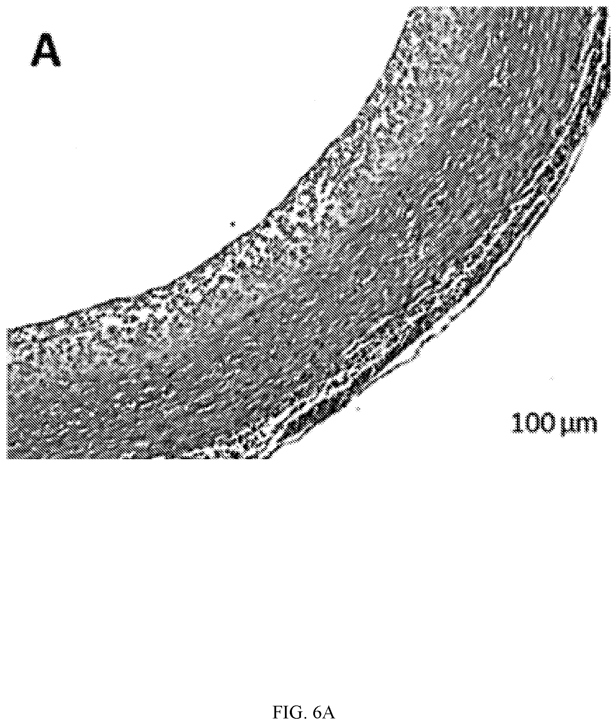

FIGs. 6A-6F illustrate histology of a scaffold formed into a vascular graft in accordance with the present disclosure, after 1 month *in vivo* in a rat model. FIGs. 6A-6F also illustrate regeneration of a new blood vessel complete with an intima, media and adventitia.
FIG. 6A illustrates staining with Masson's Trichrome, which demonstrates active ECM remodeling, collagen deposition, and physiologic remodeling.

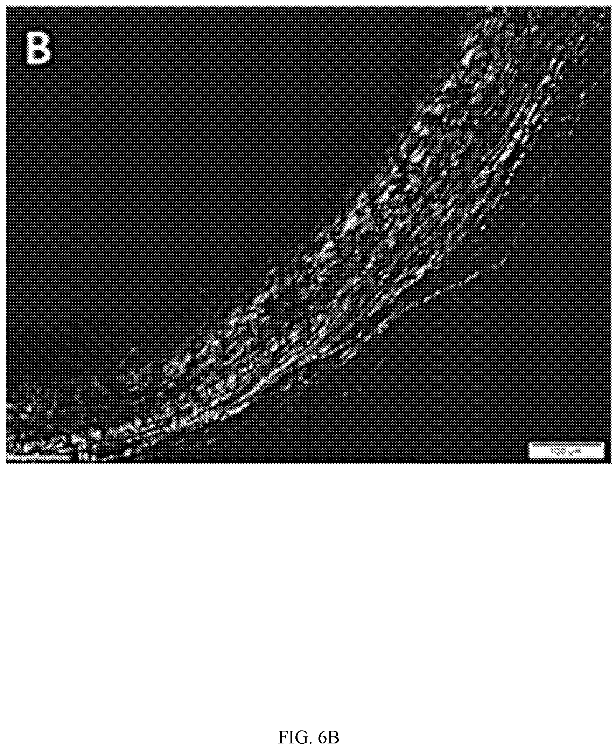

FIG. 6B is a polarized light image of picrosirius red (PCR) staining, illustrating robust collagen deposition and organization and a maturation from thin to thick fibers.

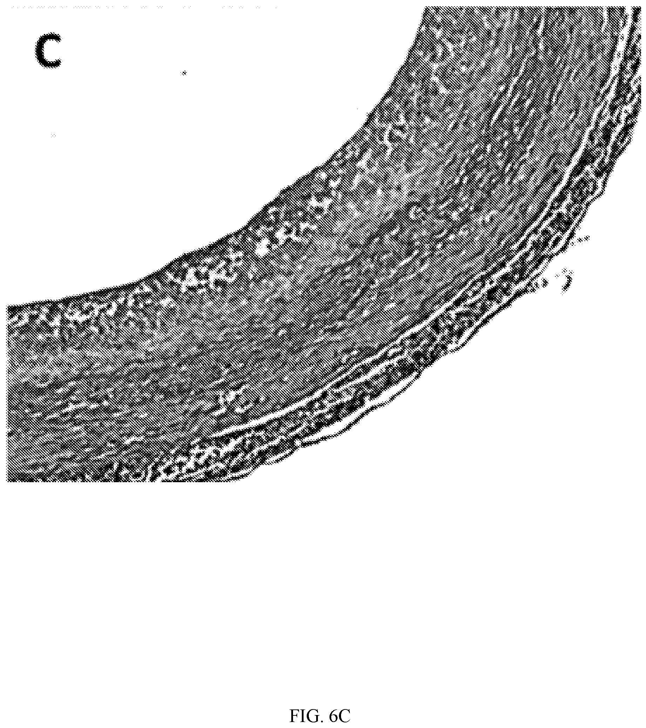

FIG. 6C illustrates Verhoeff's Van Geison (VVG) stain, demonstrating elastin deposition and organization.

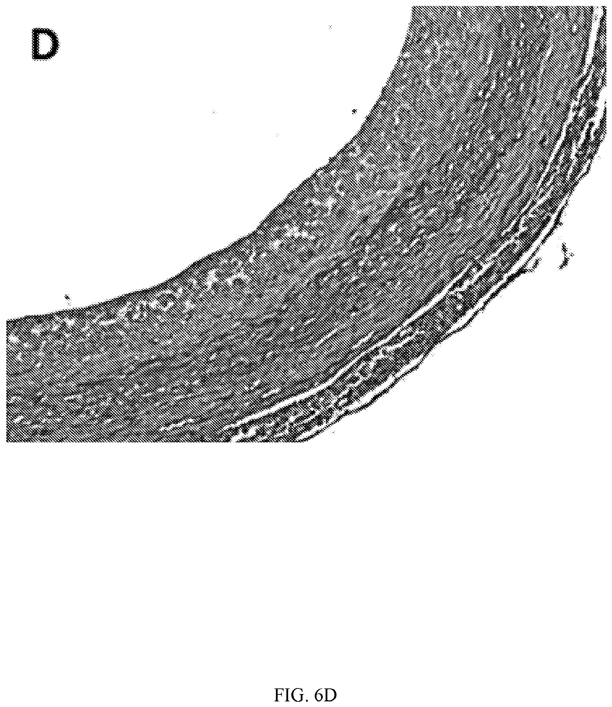

FIG. 6D illustrates Hart's staining, confirming the VVG stain results.

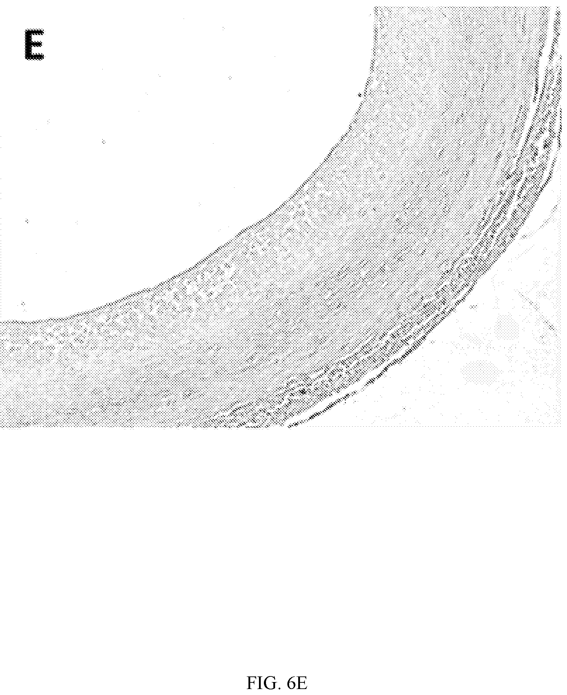

FIG. 6E illustrates Alizarin red stain for calcification, and shows no appreciable ectopic calcification.

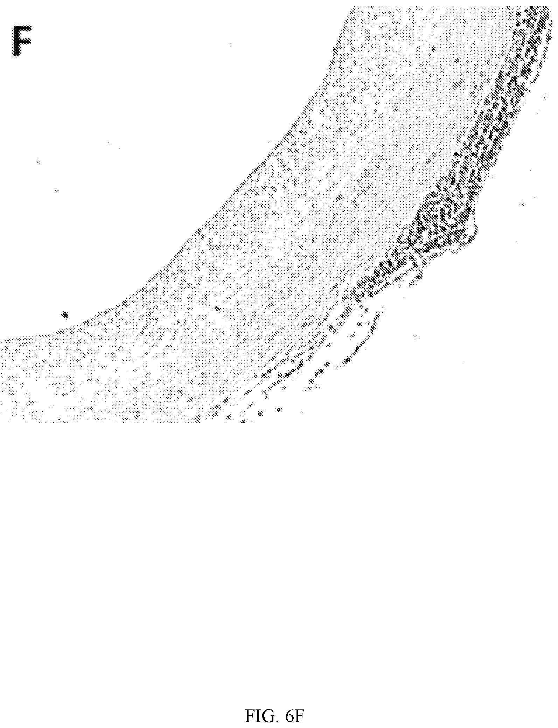

FIG. 6F illustrates Von Kossa staining, confirming the lack of calcification.

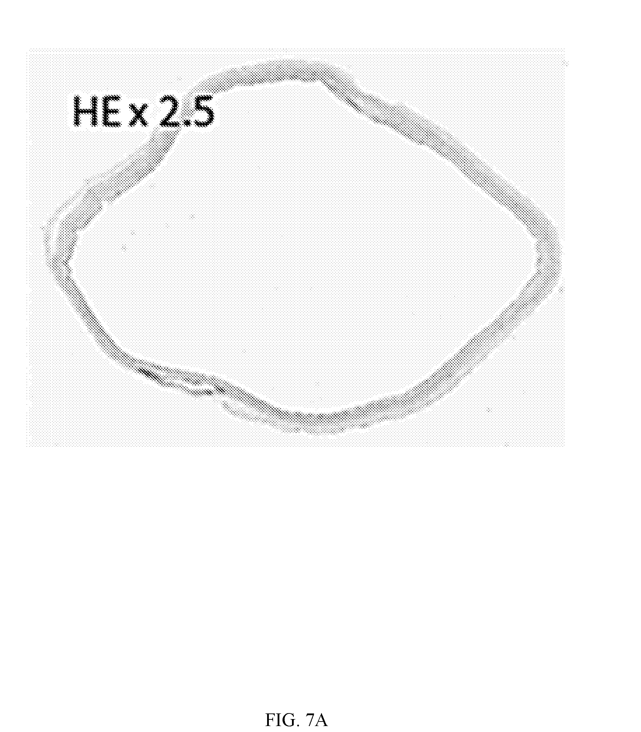

FIGs. 7A-7F illustrate histology of a native sheep inferior vena cava (IVC).
FIG. 7A illustrates H&E staining of the native IVC.

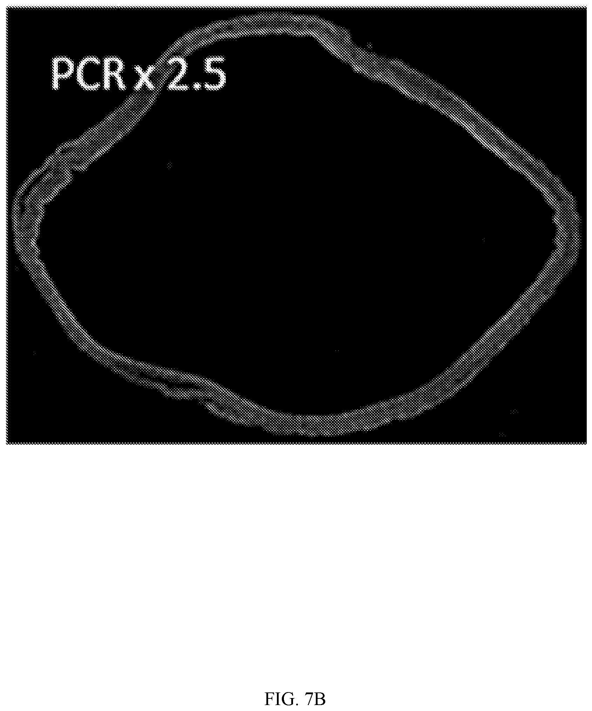

FIG. 7B illustrates PCR staining of the native IVC.

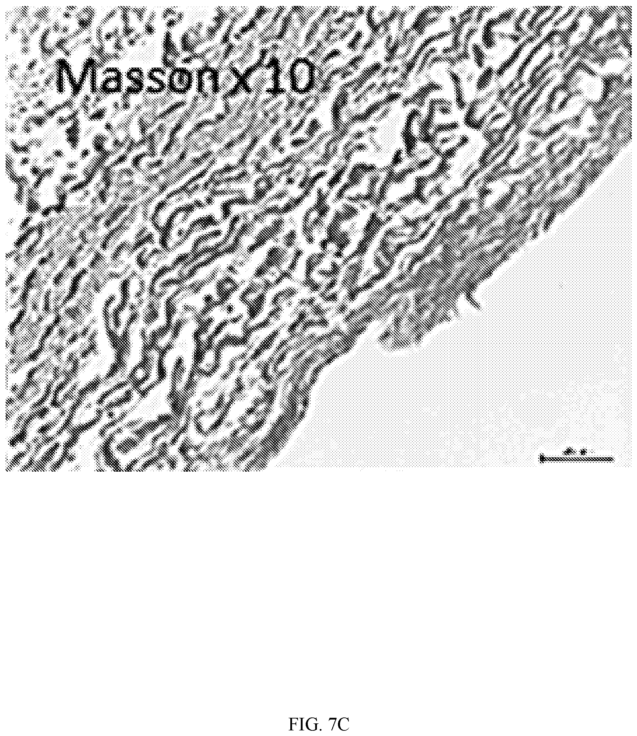

FIG. 7C illustrates Masson's Trichrome staining of the native IVC.

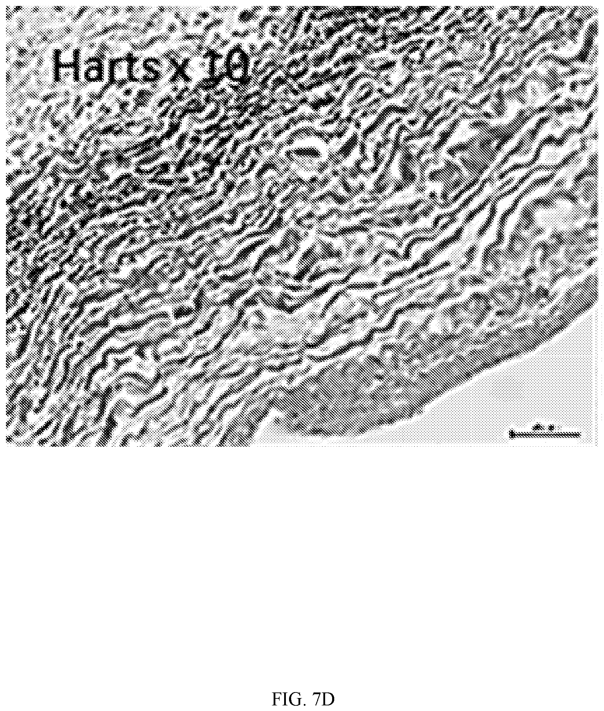

FIG. 7D illustrates Hart's staining of the native IVC.

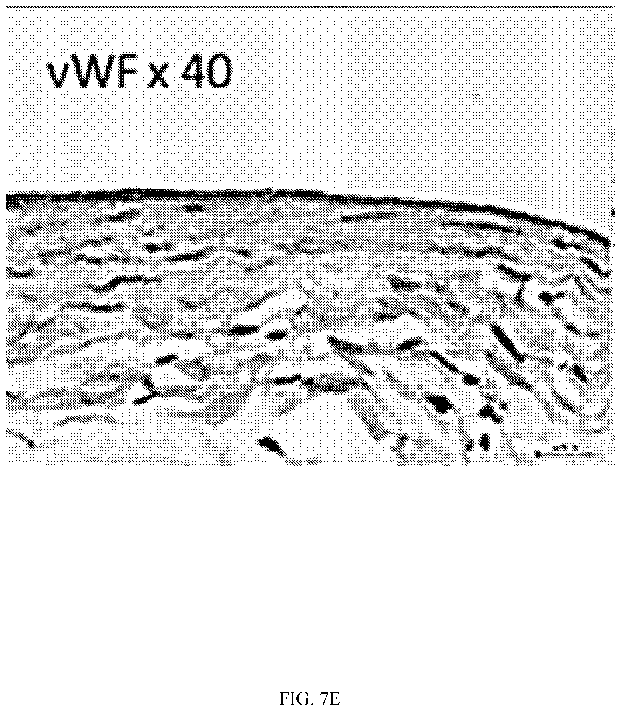

FIG. 7E illustrates Von Willebrand Factor (vWF) staining of the native IVC.

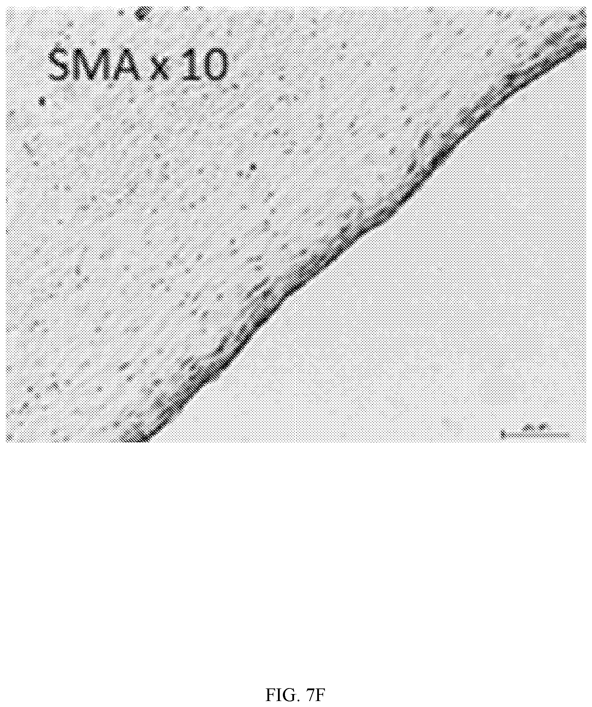

FIG. 7F illustrates smooth muscle actin (SMA) staining of the native IVC.

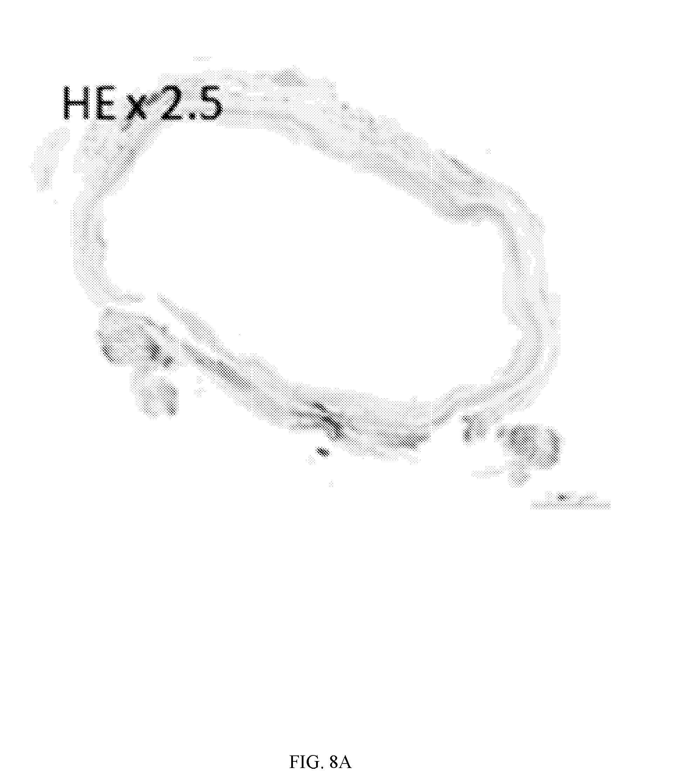

FIGs. 8A-8F illustrate histology of a scaffold formed into a vascular graft in accordance with the present disclosure, after 6 months *in vivo* in a sheep model. FIGs. 8A-8F may be compared with the histology of the native sheep IVC shown in FIGs. 7A-7F.
FIG. 8A illustrates H&E staining of the vascular graft, demonstrating polymer scaffold degradation and thin neotissue formation mimicking the native IVC.

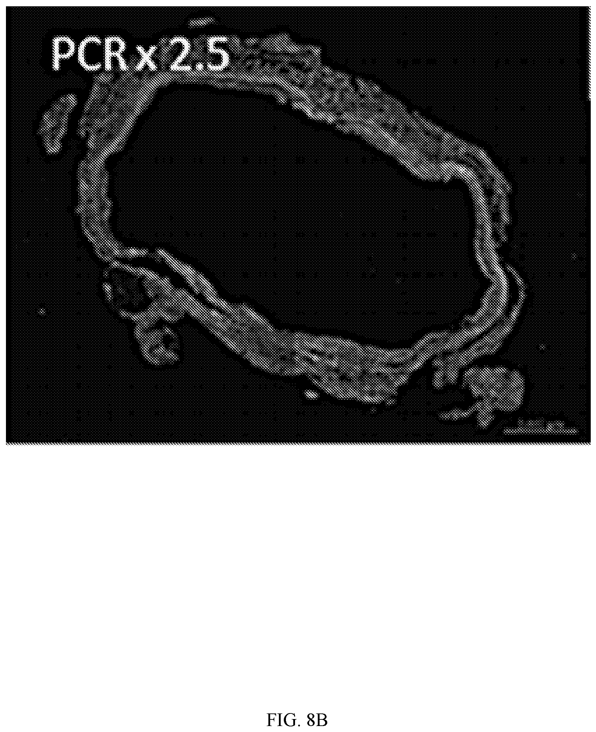

FIG. 8B illustrates PCR staining of the vascular graft, showing mature collagen formation mimicking the native IVC.

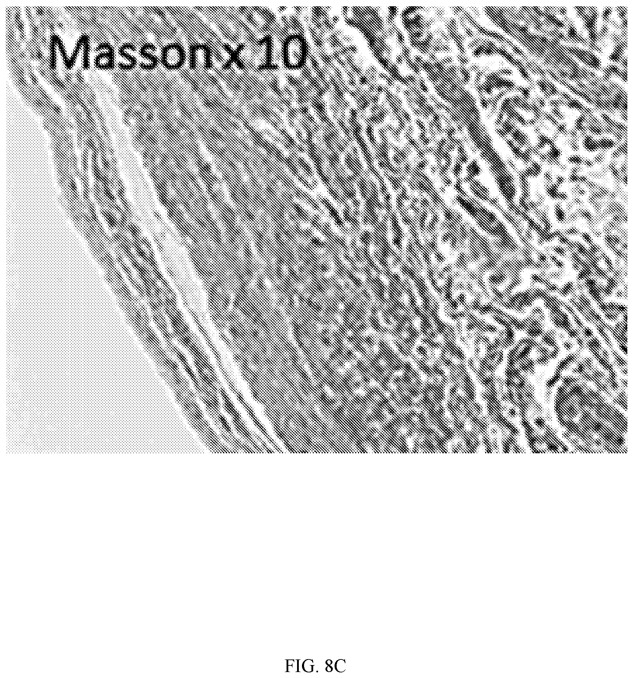

FIG. 8C illustrates Masson's Trichrome staining of the vascular graft, showing mature collagen formation mimicking the native IVC.

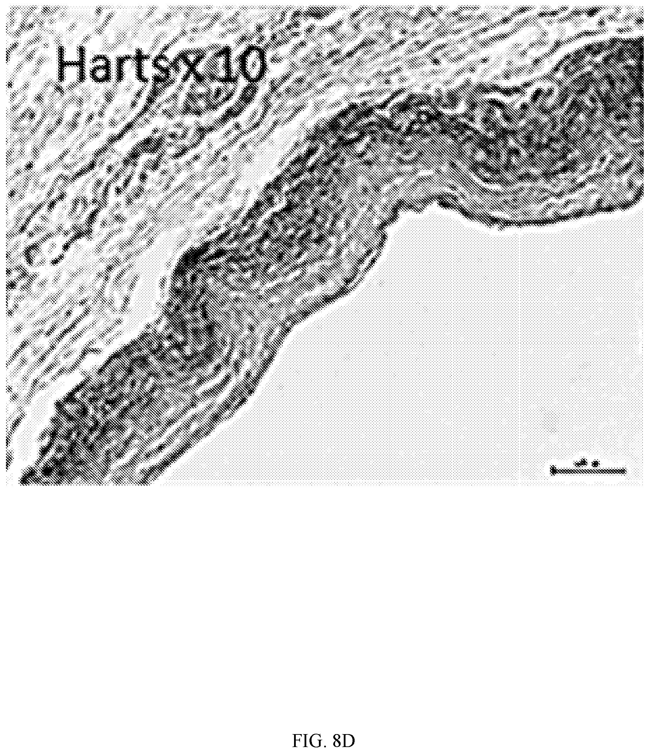

FIG. 8D illustrates Hart's staining of the vascular graft, showing oriented elastin mimicking the native IVC.

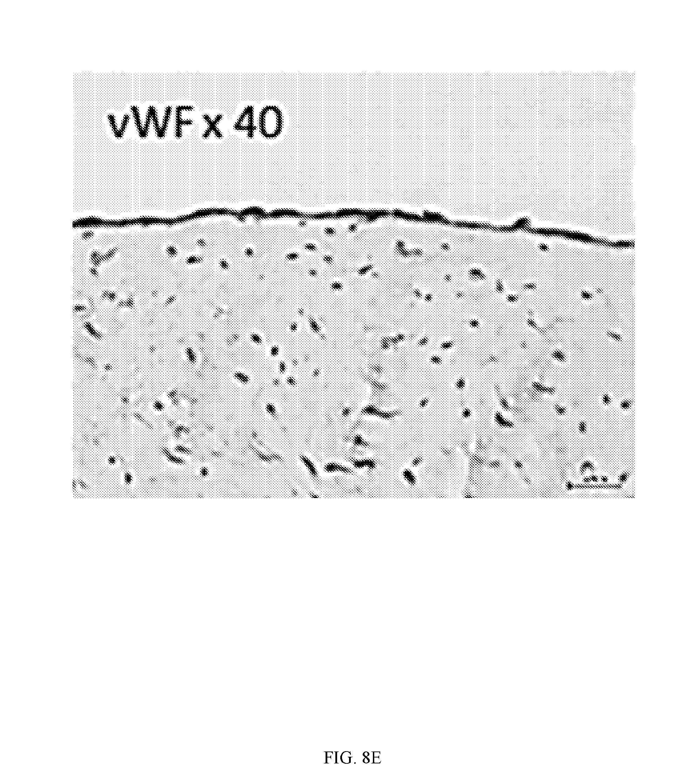

FIG. 8E illustrates vWF staining of the vascular graft, showing a complete endothelial lining.

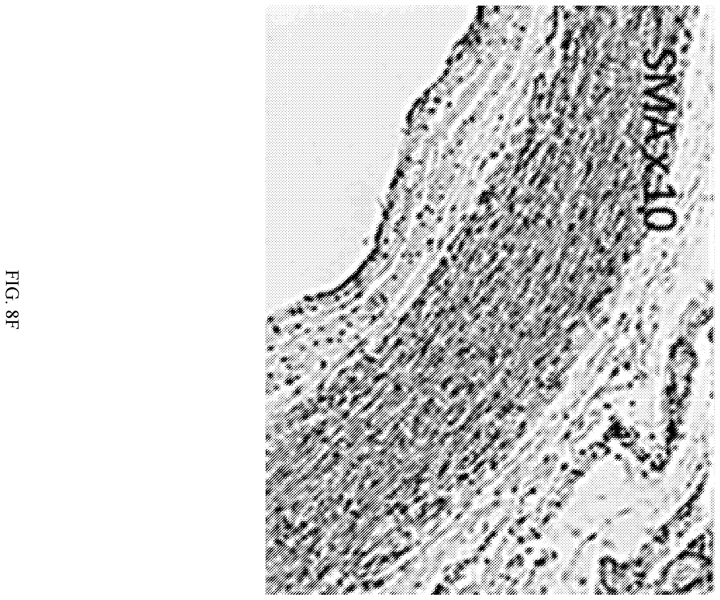

FIG. 8F illustrates SMA staining of the vascular graft, showing an oriented smooth muscle layer mimicking the native IVC.

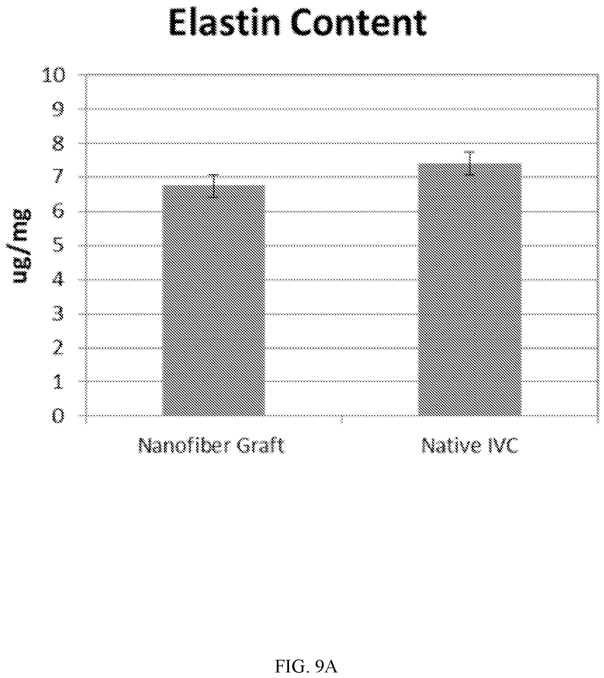

FIG. 9A illustrates a comparison of the elastin content of the native IVC and a scaffold formed into a vascular graft in accordance with the present disclosure, after 6 months *in vivo* in a sheep model.

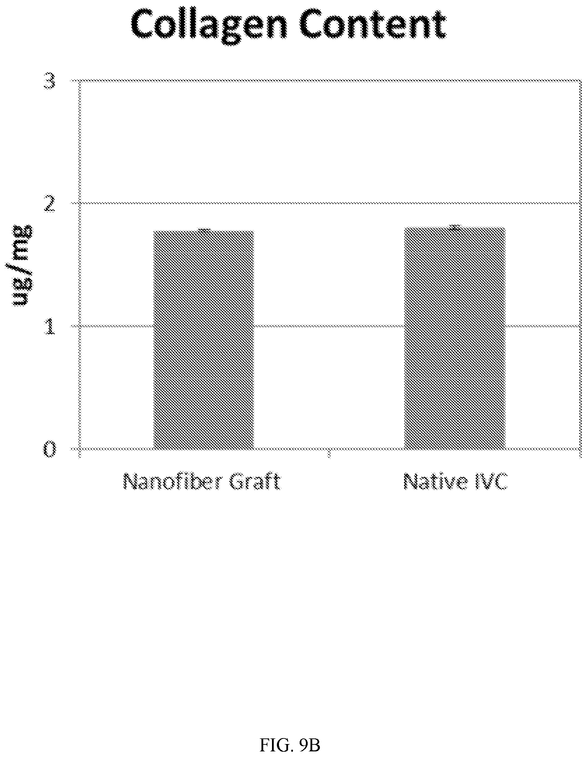

FIG. 9B illustrates a comparison of the collagen content of the native IVC and a scaffold formed into a vascular graft in accordance with the present disclosure, after 6 months *in vivo* in a sheep model. The similar elastin and collagen contents between the native IVC and the scaffold, as illustrated in FIGs. 9A and 9B, demonstrate remodeling of the scaffold into a fully functional neovessel.

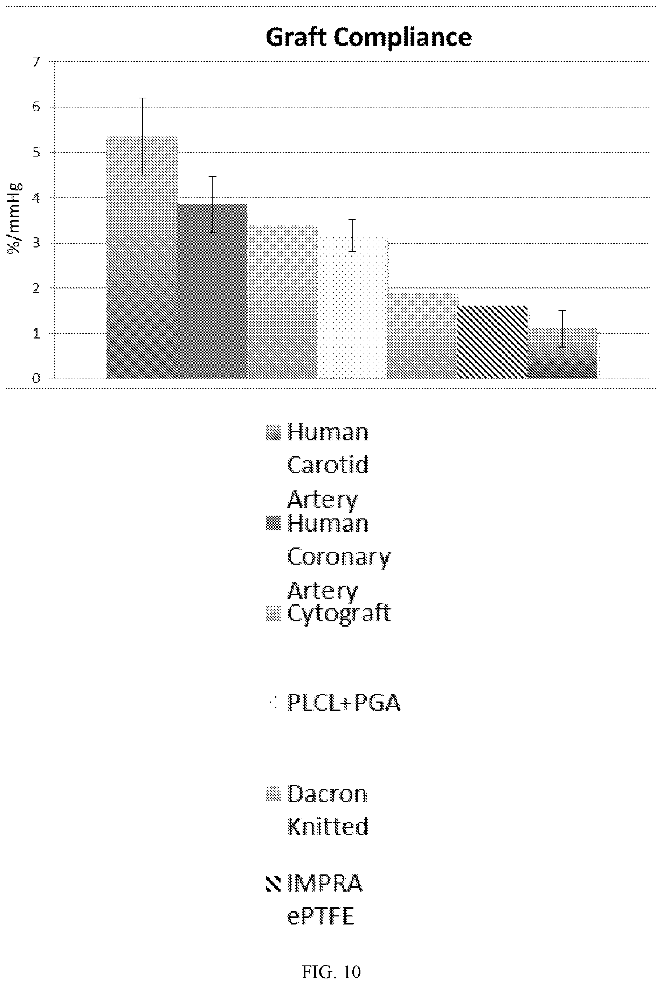

FIG. 10 illustrates compliance values in %/mmHg for a scaffold formed into a vascular graft in accordance with the present disclosure (labeled "PLCL+PGA"), in comparison with a human carotid artery and various commercially available synthetic vascular grafts. Error bars represent standard deviation.

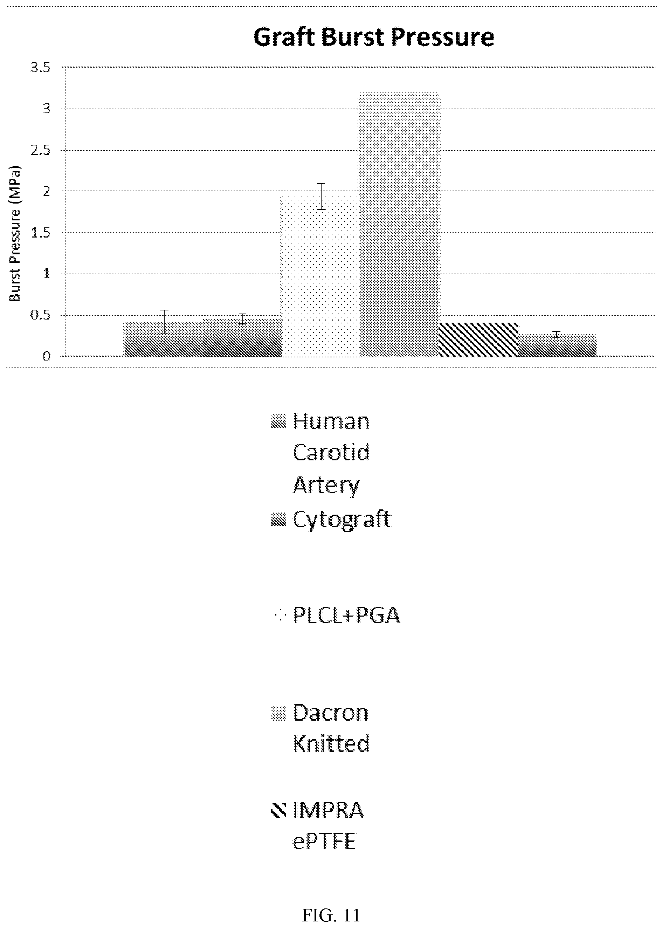

FIG. 11 illustrates burst pressure in MPa for a scaffold formed into a vascular graft in accordance with the present disclosure (labeled "PLCL+PGA"), in comparison with a human carotid artery and various commercially available synthetic vascular grafts. Error bars represent standard deviation.

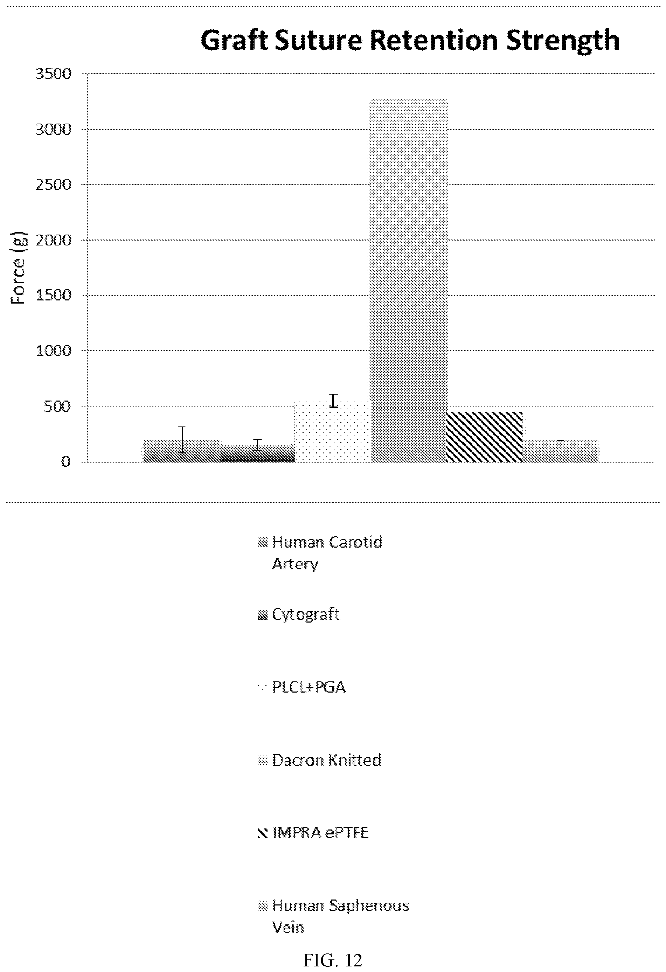

FIG. 12 illustrates graft suture retention strength in force (g) for a scaffold formed into a vascular graft in accordance with the present disclosure (labeled "PLCL+PGA"), in comparison with a human carotid artery, a human saphenous vein, and various commercially available synthetic vascular grafts. Error bars represent standard deviation.

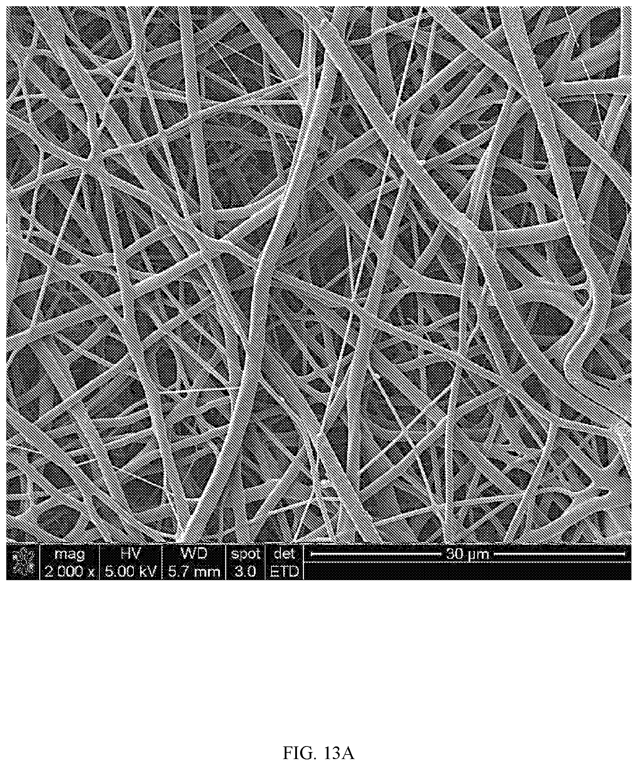

FIG. 13A is a scanning electron microscope (SEM) image of a scaffold comprising polyglycolide (PGA) and poly (lactide-co-caprolactone) (PLCL) after electrospinning and before degradation, in accordance with the present disclosure.

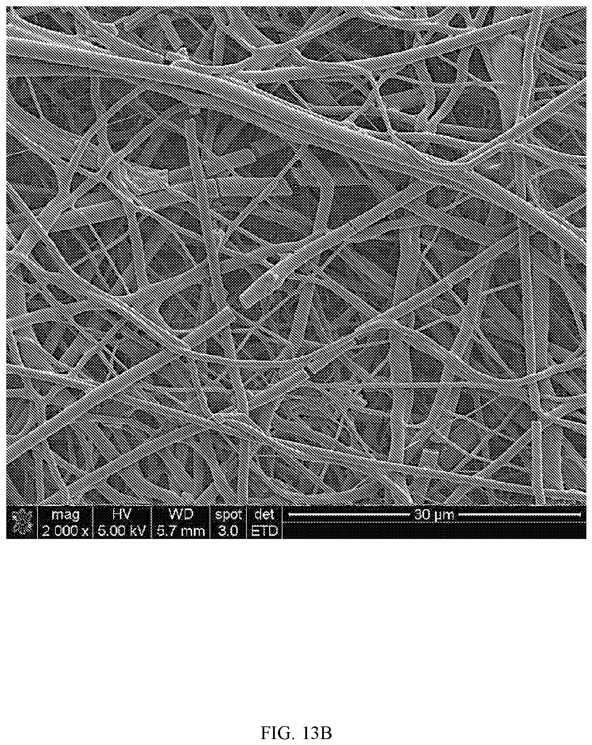

FIG. 13B illustrates the same scaffold after 1 week in PBS at 37°C, demonstrating the degradation of PGA fibers within 1 week.

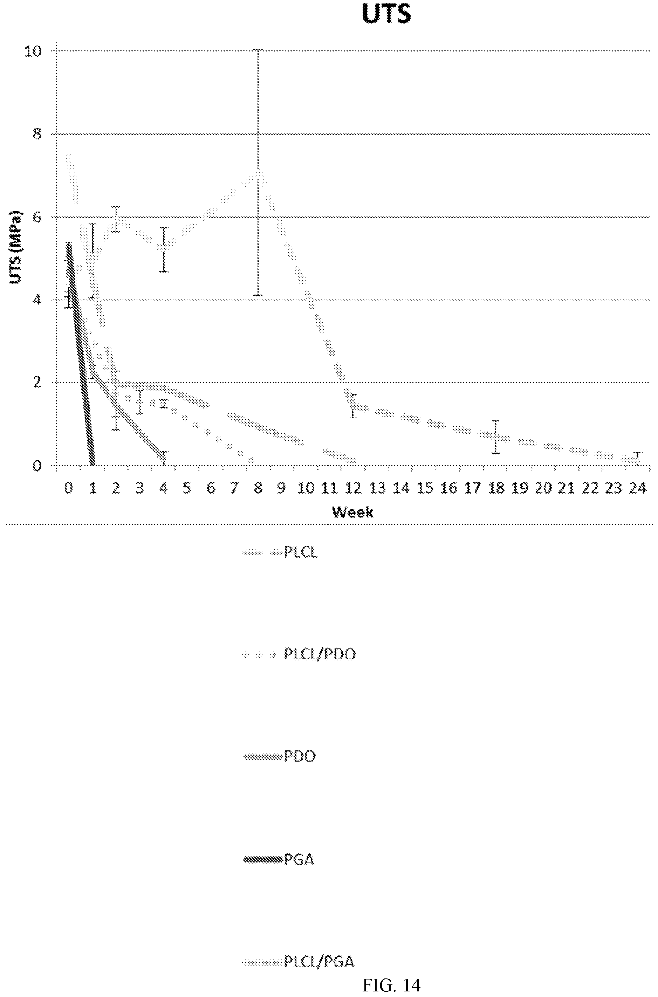

FIG. 14 illustrates the degradation of ultimate tensile strength (UTS) of fibers of various polymer combinations in PBS at 37°C. The PGA fibers degrade within about 1 week, the polydioxanone (PDO) fibers degrade within about 4 weeks, the PLCL/PDO fibers degrade within about 8 weeks, the PLCL/PGA fibers degrade within about 12 weeks, and the PLCL fibers degrade within about 24 weeks.

### DETAILED DESCRIPTION

Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described.

It must also be noted that as used herein and in the appended claims, the singular forms "a," "an," and "the" include plural references unless the context clearly dictates otherwise. Thus, for example, reference to a "fiber" is a reference to one or more fibers and equivalents thereof known to those skilled in the art, and so forth.

As used herein, the terms "about" and "approximately" mean plus or minus 10% of the numerical value of the number with which it is being used. Therefore, about 40% means in the range of 30%-50%.

The terms "animal," "patient," and "subject" as used herein include, but are not limited to, humans and non-human vertebrates such as wild, domestic and farm animals. In some embodiments, the terms "animal," "patient," and "subject" may refer to humans.

### Electrospinning fibers

Electrospinning is a method which may be used to process a polymer solution into a fiber. In embodiments wherein the diameter of the resulting fiber is on the nanometer scale, the fiber may be referred to as a nanofiber. Fibers may be formed into a variety of shapes by using a range of receiving surfaces, such as mandrels or collectors. In some embodiments, a flat shape, such as a patch, sheet, or sheet-like fiber mold or fiber scaffold, may be formed by using a substantially round or cylindrical mandrel, and cutting and unrolling the resulting fiber mold to form the sheet. The resulting fiber molds or shapes may be used in many applications, including the repair or replacement of biological structures. In some embodiments, the resulting fiber scaffold may be implanted into a biological organism or a portion thereof. In other embodiments, the resulting fiber scaffold may be placed on or affixed to a wound or a portion thereof.

Electrospinning methods may involve spinning a fiber from a polymer solution by applying a high DC voltage potential between a polymer injection system and a mandrel. In some embodiments, one or more charges may be applied to one or more components of an electrospinning system. In some embodiments, a charge may be applied to the mandrel, the polymer injection system, or combinations or portions thereof. Without wishing to be bound by theory, as the polymer solution is ejected from the polymer injection system, it is thought to be destabilized due to its exposure to a charge. The destabilized solution may then be attracted to a charged mandrel. As the destabilized solution moves from the polymer injection system to the mandrel, its solvents may evaporate and the polymer may stretch, leaving a long, thin fiber that is deposited onto the mandrel. The polymer solution may form a Taylor cone as it is ejected from the polymer injection system and exposed to a charge.

In some embodiments, more than one polymer fiber may be electrospun simultaneously onto the same mandrel from more than one polymer solution, in a process sometimes referred to as "co-spinning."

### Polymer injection system

A polymer injection system may include any system configured to eject some amount of a polymer solution into an atmosphere to permit the flow of the polymer solution from the injection system to the mandrel. In some embodiments, the polymer injection system may deliver a continuous or linear stream with a controlled volumetric flow rate of a polymer solution to be formed into a fiber. In other embodiments, the polymer injection system may deliver a variable stream of a polymer solution to be formed into a fiber. In still other embodiments, the polymer injection system may be configured to deliver intermittent streams of a polymer solution to be formed into multiple fibers. In some embodiments, the polymer injection system may include a syringe under manual or automated control. In some embodiments, the polymer injection system may include multiple syringes and multiple needles or needle-like components under individual or combined manual or automated control. In some embodiments, a multi-syringe polymer injection system may include multiple syringes and multiple needles or needle-like components, with each syringe containing the same polymer solution. In some embodiments, a multi-syringe polymer injection system may include multiple syringes and multiple needles or needle-like components, with each syringe containing a different polymer solution. In other embodiments, the polymer injection system may comprise one or more polymer injection systems, such as a first polymer injection system, a second polymer injection system, a third polymer injection system, and so on. In some embodiments, a charge may be applied to the polymer injection system, or to a portion thereof. In some embodiments, a charge may be applied to a needle or needle-like component of the polymer injection system.

In some embodiments, the polymer solution may be ejected from the polymer injection system at a flow rate of less than or equal to about 5 mL/h. In other embodiments, the polymer solution may be ejected from the polymer injection system at a flow rate in a range from about 0.01 mL/h to about 50 mL/h. The flow rate at which the polymer solution is ejected from the polymer injection system may be, in some non-limiting examples, about 0.01 mL/h, about 0.05 mL/h, about 0.1 mL/h, about 0.5 mL/h, about 1 mL/h, 2 mL/h, about 3 mL/h, about 4 mL/h, about 5 mL/h, about 6 mL/h, about 7 mL/h, about 8 mL/h, about 9 mL/h, about 10 mL/h, about 11 mL/h, about 12 mL/h, about 13 mL/h, about 14 mL/h, about 15 mL/h, about 16 mL/h, about 17 mL/h, about 18 mL/h, about 19 mL/h, about 20 mL/h, about 21 mL/h, about 22 mL/h, about 23 mL/h, about 24 mL/h, about 25 mL/h, about 26 mL/h, about 27 mL/h, about 28 mL/h, about 29 mL/h, about 30 mL/h, about 31 mL/h, about 32 mL/h, about 33 mL/h, about 34 mL/h, about 35 mL/h, about 36 mL/h, about 37 mL/h, about 38 mL/h, about 39 mL/h, about 40 mL/h, about 41 mL/h, about 42 mL/h, about 43 mL/h, about 44 mL/h, about 45 mL/h, about 46 mL/h, about 47 mL/h, about 48 mL/h, about 49 mL/h, about 50 mL/h, or any range between any two of these values, including endpoints.

As the polymer solution travels from the polymer injection system toward the mandrel, the diameter of the resulting fibers may be in the range of about 0.1µm to about 10µm. Some non-limiting examples of electrospun fiber diameters may include about 0.1µm, about 0.2µm, about 0.5µm, about 1µm, about 2µm, about 5µm, about 10µm, about 20µm, or ranges between any two of these values, including endpoints.

### Polymer solution

In some embodiments, the polymer injection system may be filled with a polymer solution. In some embodiments, the polymer solution may comprise one or more polymers. In some embodiments, the polymer solution may be a fluid formed into a polymer liquid by the application of heat. A polymer solution as defined in the second aspect of the invention comprises at least one of polylactic acid, polyglycolide, poly(lactide-co-caprolactone), or poly(lactide-co-glycolide). Other polymers include, without limitation, polyethylene terephthalate (PET), polyester, polymethylmethacrylate, polyacrylonitrile, silicone, polyurethane, polycarbonate, polyether ketone ketone, polyether ether ketone, polyether imide, polyamide, polystyrene, polyether sulfone, polysulfone, polyvinyl alcohol (PVA), polyvinyl acetate (PVAc), polycaprolactone (PCL), , polyglycerol sebacic, polydiol citrate, polyhydroxy butyrate, polyether amide, polydiaxanone (PDO), poly-L-lactide, and combinations or derivatives thereof. Alternative polymer solutions used for electrospinning may include natural polymers such as fibronectin, collagen, gelatin, hyaluronic acid, chitosan, or combinations thereof. It may be understood that polymer solutions may also include a combination of synthetic polymers and naturally occurring polymers in any combination or compositional ratio. In some non-limiting examples, the polymer solution may comprise a weight percent ratio of, for example, polyethylene terephthalate to polyurethane, from about 10% to about 90%. Non-limiting examples of such weight percent ratios may include 10%, 25%, 33%, 50%, 66%, 75%, 90%, or ranges between any two of these values, including endpoints.

In some embodiments, the polymer solution may comprise one or more solvents. In some embodiments, the solvent may comprise, for example, acetone, dimethylformamide, dimethylsulfoxide, N-methylpyrrolidone, N,N-dimethylformamide, Nacetonitrile, hexanes, ether, dioxane, ethyl acetate, pyridine, toluene, xylene, tetrahydrofuran, trifluoroacetic acid, hexafluoroisopropanol, acetic acid, dimethylacetamide, chloroform, dichloromethane, water, alcohols, ionic compounds, or combinations thereof. The concentration range of a polymer or polymers in a solvent or solvents may be, without limitation, from about 1 wt% to about 50 wt%. Some non-limiting examples of polymer concentration in solution may include about 1 wt%, 3 wt%, 5 wt%, about 10 wt%, about 15 wt%, about 20 wt%, about 25 wt%, about 30 wt%, about 35 wt%, about 40 wt%, about 45 wt%, about 50 wt%, or ranges between any two of these values, including endpoints.

In some embodiments, the polymer solution may also include additional materials. Non-limiting examples of such additional materials may include radiation opaque materials, contrast agents, electrically conductive materials, fluorescent materials, luminescent materials, antibiotics, growth factors, vitamins, cytokines, steroids, antiinflammatory drugs, small molecules, sugars, salts, peptides, proteins, cell factors, DNA, RNA, or any other materials to aid in non-invasive imaging, or any combination thereof. In some embodiments, the radiation opaque materials may include, for example, barium, tantalum, tungsten, iodine, gadolinium, gold, platinum, bismuth, or bismuth (III) oxide. In some embodiments, the electrically conductive materials may include, for example, gold, silver, iron, or polyaniline.

In some embodiments, the additional materials may be present in the polymer solution in an amount from about 1 wt% to about 500 wt%. In some non-limiting examples, the additional materials may be present in the polymer solution in an amount of about 1 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, about 100 wt%, about 125 wt%, about 150 wt%, about 175 wt%, about 200 wt%, about 225 wt%, about 250 wt%, about 275 wt%, about 300 wt%, about 325 wt%, about 350 wt%, about 375 wt%, about 400 wt%, about 425 wt%, about 450 wt%, about 475 wt%, about 500 wt%, or any range between any of these two values, including endpoints.

The type of polymer in the polymer solution may determine the characteristics of the electrospun fiber. Some fibers may be composed of polymers that are bio-stable and not absorbable or biodegradable when implanted. Such fibers may remain generally chemically unchanged for the length of time in which they remain implanted. Alternatively, fibers may be composed of polymers that may be absorbed or bio-degraded over time.

In some embodiments, a polymeric electrospun fiber may have a degradation rate. The degradation rate may be defined in a number of ways, including, for example, by the amount of time the fiber takes to degrade completely when exposed to a bodily tissue or fluid. In such embodiments, the degradation rate may be, for example, from about 1 day to about 24 months. Some non-limiting examples of degradation rates in terms of the amount of time the fiber takes to degrade completely when exposed to a bodily tissue or fluid include about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, about 30 days, about 1 month, about 1.5 months, about 2 months, about 2.5 months, about 3 months, about 3.5 months, about 4 months, about 4.5 months, about 5 months, about 5.5 months, about 6 months, about 6.5 months, about 7 months, about 7.5 months, about 8 months, about 8.5 months, about 9 months, about 9.5 months, about 10 months, about 10.5 months, about 11 months, about 11.5 months, about 12 months, about 12.5 months, about 13 months, about 13.5 months, about 14 months, about 14.5 months, about 15 months, about 15.5 months, about 16 months, about 16.5 months, about 17 months, about 17.5 months, about 18 months, about 18.5 months, about 19 months, about 19.5 months, about 20 months, about 20.5 months, about 21 months, about 21.5 months, about 22 months, about 22.5 months, about 23 months, about 23.5 months, about 24 months, or any range between any two of these values, including endpoints.

In other embodiments the degradation rate of a polymeric electrospun fiber may be defined, for example, by the amount of fiber mass lost per unit of time, such as an hour, a day, a week, or a month. In still other embodiments, the degradation rate of a polymeric electrospun fiber may be defined, for example, by the loss of strength of the fiber per unit of time. The loss of strength measured may be, for example, the ultimate tensile strength (UTS) of the fiber. In such embodiments, the degradation rate may be expressed as, for example, 25% loss of UTS in 1 week, 50% loss of UTS in 2 weeks, 100% loss of UTS in 4 weeks, and so on. For example, PGA may have a 100% loss of strength in about 1 week, PDO may have 100% loss of strength in about 4 weeks, and PLCL may have a 100% loss of strength in about 24 weeks.

The fibers disclosed herein may act as an initial template or scaffold for the repair or replacement of organs and/or tissues. These organ or tissue templates or scaffolds may degrade *in vivo* once the tissues or organs have been replaced or repaired by natural structures and cells. It may be further understood that a polymer solution and its resulting electrospun fiber(s) may be composed of more than one type of polymer, and that each polymer therein may have a specific characteristic, such as bio-stability or biodegradability at a particular degradation rate.

### Applying charges to electrospinning components

In an electrospinning system, one or more charges may be applied to one or more components, or portions of components, such as, for example, a mandrel or a polymer injection system, or portions thereof. In some embodiments, a positive charge may be applied to the polymer injection system, or portions thereof. In some embodiments, a negative charge may be applied to the polymer injection system, or portions thereof. In some embodiments, the polymer injection system, or portions thereof, may be grounded. In some embodiments, a positive charge may be applied to the mandrel, or portions thereof. In some embodiments, a negative charge may be applied to the mandrel, or portions thereof. In some embodiments, the mandrel, or portions thereof, may be grounded. In some embodiments, one or more components or portions thereof may receive the same charge. In some embodiments, one or more components, or portions thereof, may receive one or more different charges.

The charge applied to any component of the electrospinning system, or portions thereof, may be from about -15kV to about 30kV, including endpoints. In some non-limiting examples, the charge applied to any component of the electrospinning system, or portions thereof, may be about -15kV, about -10kV, about -5kV, about -3kV, about -1kV, about -0.01kV, about 0.01kV, about 1kV, about 5kV, about 10kV, about 12kV, about 15kV, about 20kV, about 25kV, about 30kV, or any range between any two of these values, including endpoints. In some embodiments, any component of the electrospinning system, or portions thereof, may be grounded.

### Mandrel movement during electrospinning

During electrospinning, in some embodiments, the mandrel may move with respect to the polymer injection system. In some embodiments, the polymer injection system may move with respect to the mandrel. The movement of one electrospinning component with respect to another electrospinning component may be, for example, substantially rotational, substantially translational, or any combination thereof. In some embodiments, one or more components of the electrospinning system may move under manual control. In some embodiments, one or more components of the electrospinning system may move under automated control. In some embodiments, the mandrel may be in contact with or mounted upon a support structure that may be moved using one or more motors or motion control systems. The pattern of the electrospun fiber deposited on the mandrel may depend upon the one or more motions of the mandrel with respect to the polymer injection system. In some embodiments, the mandrel surface may be configured to rotate about its long axis. In one non-limiting example, a mandrel having a rotation rate about its long axis that is faster than a translation rate along a linear axis, may result in a nearly helical deposition of an electrospun fiber, forming windings about the mandrel. In another example, a mandrel having a translation rate along a linear axis that is faster than a rotation rate about a rotational axis, may result in a roughly linear deposition of an electrospun fiber along a linear extent of the mandrel.

### Multi-component electrospun fiber scaffolds

In some embodiments, a scaffold comprises two or more different materials, each material having a different degradation rate as described above. In such a scaffold the degradation rate of the scaffold *in vivo* may be selectively controlled by choosing the appropriate first and second materials having the appropriate degradation rates. In some embodiments, it may be advantageous to include a first material having a relatively high or fast degradation rate, such that it degrades quickly *in vivo,* creating a scaffold porosity that encourages cellular ingrowth or infiltration into the scaffold. It may also be advantageous to include a second material having a slower or lower degradation rate than the first degradation rate, such that the second material degrades more slowly *in vivo,* and provides a scaffold to support the formation and development of new extracellular matrices by the newly infiltrated cells. Such a scaffold may be particularly advantageous for promoting wound healing.

In some embodiments, a scaffold may comprise a first polymeric electrospun fiber comprising a first material, and a second polymeric electrospun fiber comprising a second material. The first material comprises a first degradation rate, as described above, and the second material comprises a second degradation rate. The first and second degradation rates are different from each other. The first degradation rate may be, for example, higher (or faster) than the second degradation rate, or may be lower (or slower) than the second degradation rate. The degradation rate may be any rate described herein, or any equivalent known in the art. In some embodiments, the first material may have a first degradation rate of about 1 week (i.e. 100% of the first material is degraded after about 1 week *in vivo*), and the second material may have a second degradation rate of about 4 months (i.e. 100% of the second material is degraded after about 4 months *in vivo*). These degradation rates may be particularly effective in wound healing applications because the first material may degrade at a rate that is correlated with the rate at which cellular infiltration occurs (i.e. the amount of the first material lost over a given period of time is directly correlated with the amount of cellular material that infiltrates the scaffold over the same period of time). The relatively rapid degradation of the first material may create pores within the scaffold that are appropriately sized to encourage further cellular infiltration. In turn, the second material may degrade at a rate that allows it to provide structural stability to the newly infiltrating cells, remaining intact *in vivo* long enough to transfer mechanical loads in a way that encourages the infiltrated cells to form their own extracellular matrix, ultimately contributing to the long-term function of the healed tissue.

In one embodiment, the first material comprises polyglycolide (PGA), and the second material comprises poly (lactide-co-caprolactone) (PLCL). In another embodiment, the first material comprises polydioxanone (PDO), and the second material comprises PLCL. In some embodiments, PGA may be useful because it degrades *in vivo* within about 1 week, allowing cells to infiltrate the scaffold at a rate correlated with the degradation rate of the material itself. In other embodiments, PDO may be useful for similar reasons. In some embodiments, PLCL may be useful because it degrades *in vivo* within about 4 months, which allows it to provide a relatively elastic scaffold for the healing wound that may properly transfer mechanical load and force to the newly forming extracellular matrices, allowing the cells to remodel into an organized tissue structure.

In some embodiments, the first material may have a first degradation rate that is from about 2 times to about 52 times higher or faster than the second degradation rate of the second material. The first material's degradation rate may be, for example, about 2 times higher than the second material's degradation rate, about 3 times higher than the second material's degradation rate, about 4 times higher than the second material's degradation rate, about 5 times higher than the second material's degradation rate, about 6 times higher than the second material's degradation rate, about 7 times higher than the second material's degradation rate, about 8 times higher than the second material's degradation rate, about 9 times higher than the second material's degradation rate, about 10 times higher than the second material's degradation rate, about 11 times higher than the second material's degradation rate, about 12 times higher than the second material's degradation rate, about 13 times higher than the second material's degradation rate, about 14 times higher than the second material's degradation rate, about 15 times higher than the second material's degradation rate, about 16 times higher than the second material's degradation rate, about 17 times higher than the second material's degradation rate, about 18 times higher than the second material's degradation rate, about 19 times higher than the second material's degradation rate, about 20 times higher than the second material's degradation rate, about 21 times higher than the second material's degradation rate, about 22 times higher than the second material's degradation rate, about 23 times higher than the second material's degradation rate, about 24 times higher than the second material's degradation rate, about 25 times higher than the second material's degradation rate, about 26 times higher than the second material's degradation rate, about 27 times higher than the second material's degradation rate, about 28 times higher than the second material's degradation rate, about 29 times higher than the second material's degradation rate, about 30 times higher than the second material's degradation rate, about 31 times higher than the second material's degradation rate, about 32 times higher than the second material's degradation rate, about 33 times higher than the second material's degradation rate, about 34 times higher than the second material's degradation rate, about 35 times higher than the second material's degradation rate, about 36 times higher than the second material's degradation rate, about 37 times higher than the second material's degradation rate, about 38 times higher than the second material's degradation rate, about 39 times higher than the second material's degradation rate, about 40 times higher than the second material's degradation rate, about 41 times higher than the second material's degradation rate, about 42 times higher than the second material's degradation rate, about 43 times higher than the second material's degradation rate, about 44 times higher than the second material's degradation rate, about 45 times higher than the second material's degradation rate, about 46 times higher than the second material's degradation rate, about 47 times higher than the second material's degradation rate, about 48 times higher than the second material's degradation rate, about 49 times higher than the second material's degradation rate, about 50 times higher than the second material's degradation rate, about 51 times higher than the second material's degradation rate, about 52 times higher than the second material's degradation rate, or any range between any two of these values, including endpoints.

In some embodiments, the first polymeric electrospun fiber and the second polymeric electrospun fiber may be present in a weight ratio. In some examples, the weight ratio may be, for example, about 1:1, about 2:1, about 3:1, about 1:2, about 1:3, or any range between any two of these values, including endpoints. In a preferred embodiment, the weight ratio may be about 1: 1.

In certain embodiments, the first polymeric electrospun fiber may comprise a first layer of polymeric electrospun fibers, and the second polymeric electrospun fiber may comprise a second layer of polymeric electrospun fibers. In some embodiments, the scaffold disclosed herein may have multiple layers of polymeric electrospun fibers, each layer comprising a single material, or each layer comprising more than one material. In some embodiments, the first layer may be, for example, a layer comprising PGA electrospun fibers, and the second layer may be, for example, a layer comprising PLCL electrospun fibers. A scaffold may comprise any number of layers, including about 1 layer, about 2 layers, about 3 layers, about 4 layers, about 5 layers, and so on.

In some embodiments, the scaffold may further comprise at least one biological cell. In some examples, the biological cell may be a differentiated cell, a multipotent stem cell, a pluripotent stem cell, a totipotent stem cell not of human origin, an autologous cell, a syngeneic cell, an allogeneic cell, a bone marrow-derived stem cell, a cord blood stem cell, a mesenchymal cell, an embryonic stem cell, an induced pluripotent stem cell, an epithelial cell, an endothelial cell, a hematopoietic cell, an immunological cell, and any combination thereof.

As described above, in some embodiments, a scaffold may comprise a first polymeric electrospun fiber comprising a first material having a first degradation rate that substantially corresponds to a cell infiltration rate, and a second polymeric electrospun fiber comprising a second material having a second degradation rate slower than the first degradation rate. In other embodiments, a scaffold may comprise a first polymeric electrospun fiber comprising a first material having a first degradation rate that substantially corresponds to a cell infiltration rate, and a second polymeric electrospun fiber comprising a second material having a second degradation rate faster than the first degradation rate.

Described herein is a kit comprising a scaffold as described herein, and a sealable pouch. The sealable pouch may comprise one or more of a desiccant component, a foil component, and at least one flashspun high-density polyethylene fiber, such as commercially available TYVEK, for example. Any combination of these elements may be used to preserve the scaffold, particularly when the scaffold is likely to be stored, such as on a shelf or in a mobile care unit, for future use. The scaffold within the kit may be stable in ambient conditions for about two years.

A method of manufacturing a scaffold disclosed herein may comprise electrospinning a first polymer having a first degradation rate by ejecting a first polymer solution from a first polymer injection system onto a mandrel, and electrospinning a second polymer fiber having a second degradation rate different from the first degradation rate by ejecting a second polymer solution from a second polymer injection system onto the mandrel. In some embodiments, the two electrospinning steps of the method are performed simultaneously, in a process sometimes referred to as "co-spinning."

As described above, a method of improving wound healing may comprise applying to a portion of a wound a scaffold comprising a first polymeric electrospun fiber comprising a first material having a first degradation rate, and a second polymeric electrospun fiber comprising a second material having a second degradation rate different from the first degradation rate. The portion of the wound may comprise the entire wound in some cases. The method of improving wound healing may further comprise preseeding the scaffold with at least one biological cell selected from the group consisting of a differentiated cell, a multipotent stem cell, a pluripotent stem cell, a totipotent stem cell not of human origin, an autologous cell, a syngeneic cell, an allogeneic cell, a bone marrow-derived stem cell, a cord blood stem cell, a mesenchymal cell, an embryonic stem cell, an induced pluripotent stem cell, an epithelial cell, an endothelial cell, a hematopoietic cell, an immunological cell, and any combination thereof. The method of improving wound healing may further comprise soaking the scaffold in a treatment. The treatment may comprise, for example, platelet-rich plasma, bone marrow, stromal vascular fraction, any combination thereof, or any equivalent thereof known in the art. The method of improving wound healing may further comprise suturing the scaffold to the portion of the wound. In other embodiments, the method of improving wound healing may further comprise bandaging the portion of the wound after the scaffold has been applied.

### Multi-component vascular grafts

As used herein, the term "compliance" refers to a measure of deformation of a material when subjected to an applied force. In one embodiment, "compliance" may be calculated as a ratio of strain to stress of a material. In some embodiments, "compliance" may be considered as the inverse of the Young's modulus of elasticity of the material.

As used herein, the term "burst pressure" refers to an interior pressure within a closed shape, such as a tube or cylinder, that is sufficient to cause the material comprising the closed shape to burst or rupture. In some embodiments, the "burst pressure" may be the same as the ultimate tensile strength of the material comprising the closed shape.

In some embodiments, the scaffolds described herein may have the shape of a blood vessel or vascular graft. In such embodiments, the vascular graft may have a compliance, a burst pressure, and a suture retention strength.

In some embodiments, the compliance of the scaffold may be from about 2% per mmHg to about 14% per mmHg. The compliance of the scaffold may be, for example, about 2% per mmHg, about 3% per mmHg, about 4% per mmHg, about 5% per mmHg, about 6% per mmHg, about 7% per mmHg, about 8% per mmHg, about 9% per mmHg, about 10% per mmHg, about 11% per mmHg, about 12% per mmHg, about 13% per mmHg, about 14% per mmHg, or any range between any two of these values, including endpoints. In some embodiments, the compliance of the scaffold may vary throughout. In other embodiments, the compliance of the scaffold may be uniform throughout. In some embodiments, the compliance of a scaffold formed into a vascular graft having a first end and a second end may vary between the first end and the second end.

In some embodiments, the burst pressure of the scaffold may be from about 0.01MPa to about 10MPa. The burst pressure may be, for example, about 0.01MPa, about 0.05MPa, about 0.1MPa, about 0.15MPa, about 0.2MPa, about 0.25MPa, about 0.3MPa, about 0.35MPa, about 0.4MPa, about 0.45MPa, about 0.5MPa, about 0.55MPa, about 0.6MPa, about 0.65MPa, about 0.7MPa, about 0.75MPa, about 0.8MPa, about 0.85MPa, about 0.9MPa, about 0.95MPa, about 1MPa, about 1.5MPa, about 2MPa, about 2.5MPa, about 3MPa, about 3.5MPa, about 4MPa, about 4.5MPa, about 5MPa, about 5.5MPa, about 6MPa, about 6.5MPa, about 7MPa, about 7.5MPa, about 8MPa, about 8.5MPa, about 9MPa, about 9.5MPa, about 10MPa, or any range between any two of these values, including endpoints.

In some embodiments, the suture retention strength of the scaffold may be from about 100g to about 3500g. The suture retention strength may be, for example, about 100g, about 150g, about 200g, about 250g, about 300g, about 350g, about 400g, about 450g, about 500g, about 550g, about 600g, about 650g, about 700g, about 750g, about 800g, about 850g, about 900g, about 950g, about 1000g, about 1100g, about 1200g, about 1300g, about 1400g, about 1500g, about 1600g, about 1700g, about 1800g, about 1900g, about 2000g, about 2100g, about 2200g, about 2300g, about 2400g, about 2500g, about 2600g, about 2700g, about 2800g, about 2900g, about 3000g, about 3100g, about 3200g, about 3300g, about 3400g, about 3500g, or any range between any two of these values, including endpoints.

In some embodiments, the diameter, either internal or external of a scaffold formed into a vascular graft may range from about 0.01mm to about 30mm. The vascular graft diameter may be, for example, about 0.01mm, about 0.05mm, about 0.1mm, about 0.2mm, about 0.3mm, about 0.4mm, about 0.5mm, about 0.6mm, about 0.7mm, about 0.8mm, about 0.9mm, about 1mm, about 2mm, about 3mm, about 4mm, about 5mm, about 6mm, about 7mm, about 8mm, about 9mm, about 10mm, about 11mm, about 12mm, about 13mm, about 14mm, about 15mm, about 16mm, about 17mm, about 18mm, about 19mm, about 20mm, about 21mm, about 22mm, about 23mm, about 24mm, about 25mm, about 26mm, about 27mm, about 28mm, about 29mm, about 30mm, or any range between any two of these values, including endpoints.

The use of a multi-component electrospun fiber scaffold as described herein, in any shape including that of a vascular graft, may achieve three key attributes: 1) precisely controlling the degradation profile, 2) increasing the scaffold porosity as a function of time to facilitate cellular infiltration, and 3) transferring biomechanical loads to the neovessel to facilitate maturation and organization of the new extracellular matrix into fully functional organs, as shown in FIGs. 6A-6F. In a scaffold comprising PGA and PLCL in a 1:1 weight ratio, for example, the PGA fibers will degrade within about the first week *in vivo,* which provides additional porosity for infiltrating cells to move into while providing initial mechanical strength to the vascular graft. The PLCL will remain for approximately 12 weeks *in vivo,* allowing for the gradual transition of the biomechanical forces onto the newly deposited extracellular matrix, causing it to mature and organize. If the graft consisted of only PGA fibers, an anuerysm would quickly develop and the vessel would rupture within one week. If the entire graft consisted of only PLCL fibers, there would not be enough porosity to allow cells to completely infiltrate the scaffold, which would result in a chronic inflammatory response, inadequate remodeling, and stenosis. The multi-component scaffold may enhance the success of the graft.

### EXAMPLES

### Example 1: Method of manufacturing a scaffold

To create a co-spun PLCL+PGA scaffold, 10 wt% PGA was dissolved in hexafluoroisopropanol (HFIP) to form a first solution and 5 wt% PLCL was dissolved in HFIP to form a second solution. Each solution was stirred via a magnetic stir bar for at least 3 hours at room temperature. The PGA solution was placed in a first syringe and dispensed at a flow rate of about 2.5 mL/hr, and the PLCL solution was placed in a second syringe and dispensed at a flow rate of about 5 mL/hr to result in approximately 50% of the fibers being pure PGA and 50% of the fibers being pure PLCL. Both solutions were simultaneously electrospun (co-spun) onto the mandrel that was positioned 20 cm from the needle tip and rotated at 30 RPM. A +25 kV charge was applied to the syringe tip and electrospun fibers were deposited onto the mandrel until the desired wall thickness was achieved. The electrospun scaffold was then removed from the mandrel, terminally sterilized and ready for implantation. This manufacturing process may easily be scaled to any diameter and length of graft, and may be suitable for higher production volumes.

### Example 2: Porcine model

3cm x 3cm full thickness patches of skin were excised from the backs of Yorkshire pigs to create wounds. Scaffolds comprising PGA and PLCL in a 1:1 weight ratio (n=4) were trimmed to conform to the wound edges and placed into the wound beds (one scaffold in each wound bed). Control wounds were made with no scaffold treatment for comparison. Wound sites were covered with transparent adhesive wound dressings to keep the wound moist, and then wrapped with cotton gauze and protective wrap. The adhesive wound dressing and protective wrapping were removed every 7 days to image the wound, and then replaced with fresh dressings. The pigs were euthanized after 28 days, and samples were prepared for histology. Histology descriptions were graded and scored, and the percent wound closure was measured at each time point. FIGs. 2A-2F illustrate the healing of a control wound with no scaffold. FIG. 2A illustrates the wound at day 0. FIG. 2B illustrates the same wound at day 7, with 33% wound closure. FIG. 2C illustrates the same wound at day 14, with 78% wound closure. FIG. 2D illustrates the same wound at day 21, with 97% wound closure. FIG. 2E illustrates the same wound at day 28, with 100% wound closure. FIG. 2F illustrates histology of the wound at day 28. The histology showed an incomplete epidermal layer with a gap of approximately 2/3 of the wound. The scab over the wound had abundant neutrophils, and fibrous tissue under the wound was about twice as thick as the adjacent fibrous layers. Mild to moderate diffuse subcutaneous lymphocyte infiltration was present. FIGs. 3A-3F illustrate the healing of a scaffold-treated wound. FIG. 3A illustrates the wound at day 0. FIG. 3B illustrates the same wound with the same scaffold at day 7, with 28% wound closure. FIG. 3C illustrates the same wound with the same scaffold at day 14, with 96% wound closure. FIG. 3D illustrates the same wound with the same scaffold at day 21, with 100% wound closure. FIG. 3E illustrates the same wound with the same scaffold at day 28, with 100% wound closure. FIG. 3F illustrates histology of the wound with the scaffold at day 28. The histology showed a complete epidermal layer over the wound site with normal thickness and keratin production. Fibrous tissue under the wound was about twice as thick as the adjacent fibrous layers. Mild to moderate diffuse subcutaneous lymphocyte infiltration was present. The scaffold-treated wounds achieved complete or substantially complete wound closure about 1 week before the control wounds.

### Example 3: Field use in traumatic equine injuries

The scaffolds described herein have been used successfully by veterinarians in a variety of applications across domestic species, including dogs, cats, horses, and reptiles. FIGs. 4A-4F and 5A-5F illustrate the scaffold-enhanced healing of two horses with traumatic injuries. In both cases, a scaffold comprising PGA and PLCL in a 1:1 weight ratio was used without any additional soaking in treatment or seeding of cells.

FIGs. 4A-4F illustrate the healing process of a horse with a severe leg injury cause by a high-tensile wire. FIG. 4A illustrates the initial high-tensile wire wounds on the pastern and cannon bone. FIG. 4B illustrates both wounds fully healed 5 weeks after applying a scaffold in accordance with the present disclosure. FIG. 4C illustrates the vascular wound to the pastern at the time of injury. FIG. 4D illustrates the same vascular wound to the pastern, covered with a scaffold in accordance with the present disclosure. FIG. 4E illustrates a paraffin gauze bandage over the scaffold covering the pastern wound, in accordance with the present disclosure. FIG. 4F illustrates the pastern wound healing 4 weeks after the scaffold was applied.

FIGs. 5A-5E illustrate the healing process of a horse with a large shoulder and torso laceration caused by running into a hay rake. FIG. 5A illustrates the laceration at the time of injury. FIG. 5B illustrates the laceration at the time of injury, with a scaffold applied to the wound in accordance with the present disclosure. FIG. 5C illustrates the wound after 4 weeks of healing, with evidence of granulation tissue regrowth. FIG. 5D illustrates the wound after 2 months (8 weeks) of healing, with evidence of epithelialization and hair regrowth. FIG. 5E illustrates the wound after 5 months (20 weeks) of healing, with further epithelialization and hair regrowth.

### Example 4: Comparative mechanical testing of vascular grafts

Commercially available vascular grafts have not demonstrated a compliance that matched that of the human carotid artery. The average compliance of the native human carotid artery (5.4%/mmHg) is approximately 2x greater than that of the most compliant commercially available vascular graft (bovine heterograft 2.6%/mmHg), as shown in FIG. 10. Similarly, the average compliance of the human coronary artery (3.8%/mmHg) is approximately 1.5x that of the most compliant commercially available graft. This compliance mismatch may be a major contributing factor in the poor clinical performance of such grafts. A vascular graft in accordance with the present disclosure had a compliance of about 3.2%/mmHg, closely mimicking the human coronary artery, as shown in FIG. 10.

The burst pressure of a vascular graft in accordance with the present disclosure is well above those seen in native human saphenous veins and carotid arteries, as shown in FIG. 11. This suggests that it may be possible to adjust the sidewall thickness to tailor the graft compliance (i.e. a thinner sidewall has a higher compliance), as long as the minimum burst pressure is achieved. In contrast, the tissue engineered graft and ePTFE graft barely reach the burst pressures of the native vein and artery.

A vascular graft in accordance with the present disclosure surpassed the 2N or 204g standard minimum suture retention strength recommended for potential vascular prosthetics, as shown in FIG. 12. Suture retention strength is an important quality because the duration of surgery and risk of complications to the patient may increase if the sutures tear through the vascular graft during anastomosis *in vivo.*

### Example 5: Vascular grafts in an ovine model

Seven juvenile sheep underwent inferior vena cava (IVC) and carotid artery grafting procedures with vascular grafts made in accordance with the present disclosure. Following surgical grafting, the animals were allowed to recover for 14 days, and were followed for 6 months. The *in vivo* patency of the grafts was evaluated at 3 months and 6 months post-implantation. At each follow-up evaluation, the animals underwent venous blood sampling, veterinary examinations, and imaging to assess the integrity of the vessel grafts. In order to assess the *in vivo* luminal and longitudinal growth of the grafts, both echocardiographic and fluoroscopic angiography were performed with the animals anesthetized. One animal died 3 months after surgery, and showed occlusion in both the IVC and the carotid artery. The remaining animals were euthanized after 6 months, and underwent gross pathological evaluation and tissue harvesting. The grafted regions of the inferior vena cava and carotid artery were visually evaluated for tissue ingrowth, evidence of fibrosis, and clotting. The collected tissues were then preserved for histology. Detailed histological assessments were performed as shown in FIGs. 7A-F and 8A-F. FIGs. 9A and 9B illustrate the similar elastin and collagen contents, respectively, of the native IVC and the grafted vessel at the conclusion of this study.

### Example 6: Field use in a traumatic tortoise shell injury

An adult Gopher tortoise was presented to the Animal Veterinary Hospital of Orlando after being hit by a car. The dorsal aspect of the carapace was traumatically removed during the accident, and the deepest part of the injury extended to the surface of the pleural membrane. The turtle was admitted to the hospital for shell repair treatments and rehabilitation. The wound was cleaned and debrided. A scaffold comprising PGA and PLCL in a 1:1 weight ratio was applied to the affected area for 2 weeks. After 2 weeks, the patch was removed and noticeable shell regrowth was observed. Unaffected margins of the patch were trimmed, and the sections were reapplied to the damaged area for an additional 2 weeks. Treating veterinarian Bruce Bogoslavsky, DVM, stated, "[i]n my 27 years of veterinary practice, working with reptiles, I have never seen this rapid of a regrowth of damaged shell and can only attribute the results to the use of the [scaffold]."

## Claims

1. An electrospun polymer sheet comprising:
a plurality of fibers co-electrospun from a solution comprising a first polymer and a second polymer;
wherein the first polymer and the second polymer comprise at least one of polylactic acid, polyglycolide, poly(lactide-co-caprolactone), or poly(lactide-co-glycolide);
wherein the first polymer comprises a first degradation rate when exposed to a bodily tissue or a fluid and the second polymer comprises a second degradation rate when exposed to the bodily tissue or the fluid, wherein the second degradation rate differs from the first degradation rate;
wherein degradation of the first polymer causes the electrospun polymer sheet to form a scaffold configured to support cellular ingrowth into the scaffold.

2. The electrospun polymer sheet of claim 1, wherein the first degradation rate is about 1 week and the second degradation rate is about 4 months.

3. The electrospun polymer sheet of claim 1 or claim 2, wherein the electrospun polymer sheet comprises a single layer of the plurality of fibers.

4. The electrospun polymer sheet of any one of claims 1-3, wherein the electrospun polymer sheet comprises a plurality of layers of the plurality of fibers.

5. The electrospun polymer sheet of any one of claims 1-4, further comprising a biological cell.

6. The electrospun polymer sheet of claim 5, wherein the biological cell is selected from the group consisting of differentiated cell, a multipotent stem cell, a pluripotent stem cell, a totipotent stem cell not of human origin, an autologous cell, a syngeneic cell, an allogeneic cell, a bone marrow-derived stem cell, a cord blood stem cell, a mesenchymal cell, an embryonic stem cell, an induced pluripotent stem cell, an epithelial cell, an endothelial cell, a hematopoietic cell, an immunological cell, and any combination thereof.

7. The electrospun polymer sheet of any one of claims 1-6, wherein at least one of the first degradation rate or the second degradation rate corresponds to a cellular infiltration rate.

8. A method of making an electrospun polymer sheet, the method comprising:
preparing a first solution comprising a first polymer;
preparing a second solution comprising a second polymer, wherein the first polymer and the second polymer comprise at least one of polylactic acid, polyglycolide, poly(lactide-co-caprolactone), or poly(lactide-co-glycolide), wherein the first polymer comprises a first degradation rate when exposed to a bodily tissue or a fluid and the second polymer comprises a second degradation rate when exposed to the bodily tissue or the fluid, wherein the second degradation rate differs from the first degradation rate;
applying a charge to one or more of a receiving surface, a polymer injection system, and a solution ejected from the polymer injection system; and
simultaneously ejecting, via the polymer injection system, the first solution and the second solution onto the receiving surface to form the electrospun polymer sheet comprising a plurality of fibers, wherein the electrospun polymer sheet is configured such that degradation of the first polymer causes the electrospun polymer sheet to form a scaffold configured to support cellular ingrowth into the scaffold.

9. The method of claim 8, wherein the first degradation rate is about 1 week and the second degradation rate is about 4 months.

10. The method of claim 8 or claim 9, wherein the electrospun polymer sheet comprises a single layer of the plurality of fibers.

11. The method of any one of claims 8-10, wherein the electrospun polymer sheet comprises a plurality of layers of the plurality of fibers.

12. The method of any one of claims 8-11, further comprising a biological cell.

13. The method of claim 12, wherein the biological cell is selected from the group consisting of differentiated cell, a multipotent stem cell, a pluripotent stem cell, a totipotent stem cell not of human origin, an autologous cell, a syngeneic cell, an allogeneic cell, a bone marrow-derived stem cell, a cord blood stem cell, a mesenchymal cell, an embryonic stem cell, an induced pluripotent stem cell, an epithelial cell, an endothelial cell, a hematopoietic cell, an immunological cell, and any combination thereof.

14. The method of any one of claims 8-13, wherein at least one of the first degradation rate or the second degradation rate corresponds to a cellular infiltration rate.

## Patentansprüche

1. Elektrogesponnene Polymerfolie, umfassend:
eine Vielzahl von Fasern, die aus einer Lösung co-elektrogesponnen werden, die ein erstes Polymer und ein zweites Polymer umfasst;
wobei das erste Polymer und das zweite Polymer mindestens eines von Polymilchsäure, Polyglycolid, Poly(lactid-co-caprolacton) oder Poly(lactid-co-glycolid) umfassen;
wobei das erste Polymer eine erste Abbaurate umfasst, wenn es einem Körpergewebe oder einer Flüssigkeit ausgesetzt ist, und das zweite Polymer eine zweite Abbaurate umfasst, wenn es dem Körpergewebe oder der Flüssigkeit ausgesetzt ist, wobei sich die zweite Abbaurate von der ersten Abbaurate unterscheidet;
wobei der Abbau des ersten Polymers bewirkt, dass die elektrogesponnene Polymerfolie ein Gerüst ausbildet, das dazu konfiguriert ist, Zelleinwachsen in das Gerüst zu unterstützen.

2. Elektrogesponnene Polymerfolie nach Anspruch 1, wobei die erste Abbaurate etwa 1 Woche beträgt und die zweite Abbaurate etwa 4 Monate beträgt.

3. Elektrogesponnene Polymerfolie nach Anspruch 1 oder Anspruch 2, wobei die elektrogesponnene Polymerfolie eine einzelne Schicht der Vielzahl von Fasern umfasst.

4. Elektrogesponnene Polymerfolie nach einem der Ansprüche 1 - 3, wobei die elektrogesponnene Polymerfolie eine Vielzahl von Schichten der Vielzahl von Fasern umfasst.

5. Elektrogesponnene Polymerfolie nach einem der Ansprüche 1 - 4, ferner umfassend eine biologische Zelle.

6. Elektrogesponnene Polymerfolie nach Anspruch 5, wobei die biologische Zelle ausgewählt ist aus der Gruppe bestehend aus differenzierter Zelle, einer multipotenten Stammzelle, einer pluripotenten Stammzelle, einer totipotenten Stammzelle nicht menschlichen Ursprungs, einer autologen Zelle, einer syngenen Zelle, einer allogenen Zelle, einer von Knochenmark abgeleiteten Stammzelle, einer Nabelschnurblutstammzelle, einer mesenchymalen Zelle, einer embryonalen Stammzelle, einer induzierten pluripotenten Stammzelle, einer Epithelzelle, einer Endothelzelle, einer hämatopoetischen Zelle, einer immunologischen Zelle und einer beliebigen Kombination davon.

7. Elektrogesponnene Polymerfolie nach einem der Ansprüche 1-6, wobei mindestens eine von der ersten Abbaurate oder der zweiten Abbaurate einer Zellinfiltrationsrate entspricht.

8. Verfahren zum Herstellen einer elektrogesponnenen Polymerfolie, wobei das Verfahren Folgendes umfasst:
Zubereiten einer ersten Lösung, die ein erstes Polymer umfasst;
Zubereiten einer zweiten Lösung, die ein zweites Polymer umfasst, wobei das erste Polymer und das zweite Polymer mindestens eines von Polymilchsäure, Polyglycolid, Poly(lactid-co-caprolacton) oder Poly(lactid-co-glycolid) umfassen, wobei das erste Polymer eine erste Abbaurate umfasst, wenn es einem Körpergewebe oder einer Flüssigkeit ausgesetzt ist, und das zweite Polymer eine zweite Abbaurate umfasst, wenn es dem Körpergewebe oder der Flüssigkeit ausgesetzt ist, wobei sich die zweite Abbaurate von der ersten Abbaurate unterscheidet;
Anlegen einer Ladung an eines oder mehrere von einer Aufnahmeoberfläche, einem Polymerinjektionssystem und einer aus dem Polymerinjektionssystem ausgestoßenen Lösung; und
gleichzeitiges Ausstoßen der ersten Lösung und der zweiten Lösung über das Polymerinjektionssystem auf die Aufnahmeoberfläche, um die elektrogesponnene Polymerfolie auszubilden, die eine Vielzahl von Fasern umfasst, wobei die elektrogesponnene Polymerfolie derart konfiguriert ist, dass der Abbau des ersten Polymers bewirkt, dass die elektrogesponnene Polymerfolie ein Gerüst ausbildet, das dazu konfiguriert ist, Zelleinwachsen in das Gerüst zu unterstützen.

9. Verfahren nach Anspruch 8, wobei die erste Abbaurate etwa 1 Woche beträgt und die zweite Abbaurate etwa 4 Monate beträgt.

10. Verfahren nach Anspruch 8 oder Anspruch 9, wobei die elektrogesponnene Polymerfolie eine einzelne Schicht der Vielzahl von Fasern umfasst.

11. Verfahren nach einem der Ansprüche 8-10, wobei die elektrogesponnene Polymerfolie eine Vielzahl von Schichten der Vielzahl von Fasern umfasst.

12. Verfahren nach einem der Ansprüche 8-11, ferner umfassend eine biologische Zelle.

13. Verfahren nach Anspruch 12, wobei die biologische Zelle ausgewählt ist aus der Gruppe bestehend aus differenzierter Zelle, einer multipotenten Stammzelle, einer pluripotenten Stammzelle, einer totipotenten Stammzelle nicht menschlichen Ursprungs, einer autologen Zelle, einer syngenen Zelle, einer allogenen Zelle, einer von Knochenmark abgeleiteten Stammzelle, einer Nabelschnurblutstammzelle, einer mesenchymalen Zelle, einer embryonalen Stammzelle, einer induzierten pluripotenten Stammzelle, einer Epithelzelle, einer Endothelzelle, einer hämatopoetischen Zelle, einer immunologischen Zelle und einer beliebigen Kombination davon.

14. Verfahren nach einem der Ansprüche 8-13, wobei mindestens eine von der ersten Abbaurate oder der zweiten Abbaurate einer Zellinfiltrationsrate entspricht.

## Revendications

1. Feuille polymère électrofilée comprenant :
une pluralité de fibres co-électrofilées à partir d'une solution comprenant un premier polymère et un second polymère ;
dans laquelle le premier polymère et le second polymère comprennent au moins l'un parmi l'acide polylactique, le polyglycolide, le poly(lactide-co-caprolactone) ou le poly(lactide-co-glycolide) ;
dans laquelle le premier polymère comprend une première vitesse de dégradation lorsqu'il est exposé à un tissu ou un fluide corporel et le second polymère comprend une seconde vitesse de dégradation lorsqu'il est exposé au tissu ou fluide corporel, dans lequel la seconde vitesse de dégradation diffère de la première vitesse de dégradation ;
dans laquelle la dégradation du premier polymère amène la feuille polymère électrofilée à former une matrice conçue pour favoriser la croissance cellulaire dans la matrice.

2. Feuille polymère électrofilée selon la revendication 1, dans laquelle la première vitesse de dégradation est d'environ 1 semaine et la seconde vitesse de dégradation est d'environ 4 mois.

3. Feuille polymère électrofilée selon la revendication 1 ou la revendication 2, dans laquelle la feuille polymère électrofilée comprend une seule couche de la pluralité de fibres.

4. Feuille polymère électrofilée selon l'une quelconque des revendications 1 à 3, dans laquelle la feuille polymère électrofilée comprend une pluralité de couches de la pluralité de fibres.

5. Feuille polymère électrofilée selon l'une quelconque des revendications 1 à 4, comprenant en outre une cellule biologique.

6. Feuille polymère électrofilée selon la revendication 5, dans laquelle la cellule biologique est choisie dans le groupe constitué par une cellule différenciée, une cellule souche multipotente, une cellule souche pluripotente, une cellule souche totipotente non d'origine humaine, une cellule autologue, une cellule syngénique, une cellule allogénique, une cellule souche dérivée de la moelle osseuse, une cellule souche de sang de cordon, une cellule mésenchymateuse, une cellule souche embryonnaire, une cellule souche pluripotente induite, une cellule épithéliale, une cellule endothéliale, une cellule hématopoïétique, une cellule immunologique, et toute combinaison de celles-ci.

7. Feuille polymère électrofilée selon l'une quelconque des revendications 1 à 6, dans laquelle au moins l'une de la première vitesse de dégradation ou de la seconde vitesse de dégradation correspond à une vitesse d'infiltration cellulaire.

8. Procédé de fabrication d'une feuille polymère électrofilée, le procédé comprenant :
la préparation d'une première solution comprenant un premier polymère ;
la préparation d'une seconde solution comprenant un second polymère, dans lequel le premier polymère et le second polymère comprennent au moins l'un parmi l'acide polylactique, le polyglycolide, le poly(lactide-co-caprolactone) ou le poly(lactide-co-glycolide), dans lequel le premier polymère comprend une première vitesse de dégradation lorsqu'il est exposé à un tissu ou un fluide corporel et le second polymère comprend une seconde vitesse de dégradation lorsqu'il est exposé au tissu ou fluide corporel, dans lequel la seconde vitesse de dégradation diffère de la première vitesse de dégradation ;
l'application d'une charge sur une ou plusieurs d'une surface de réception, d'un système d'injection de polymère et d'une solution éjectée du système d'injection de polymère ; et
l'éjection simultanée, via le système d'injection de polymère, de la première solution et de la seconde solution sur la surface de réception pour former la feuille polymère électrofilée comprenant une pluralité de fibres, dans lequel la feuille polymère électrofilée est configurée de telle sorte que la dégradation du premier polymère amène la feuille polymère électrofilée à former une matrice configurée pour favoriser la croissance cellulaire dans la matrice.

9. Procédé selon la revendication 8, dans lequel la première vitesse de dégradation est d'environ 1 semaine et la seconde vitesse de dégradation est d'environ 4 mois.

10. Procédé selon la revendication 8 ou la revendication 9, dans lequel la feuille polymère électrofilée comprend une seule couche de la pluralité de fibres.

11. Procédé selon l'une quelconque des revendications 8 à 10, dans lequel la feuille polymère électrofilée comprend une pluralité de couches de la pluralité de fibres.

12. Procédé selon l'une quelconque des revendications 8 à 11, comprenant en outre une cellule biologique.

13. Procédé selon la revendication 12, dans lequel la cellule biologique est choisie dans le groupe constitué par une cellule différenciée, une cellule souche multipotente, une cellule souche pluripotente, une cellule souche totipotente non d'origine humaine, une cellule autologue, une cellule syngénique, une cellule allogénique, une cellule souche dérivée de la moelle osseuse, une cellule souche de sang de cordon, une cellule mésenchymateuse, une cellule souche embryonnaire, une cellule souche pluripotente induite, une cellule épithéliale, une cellule endothéliale, une cellule hématopoïétique, une cellule immunologique, et toute combinaison de celles-ci.

14. Procédé selon l'une quelconque des revendications 8 à 13, dans lequel au moins l'une de la première vitesse de dégradation ou de la seconde vitesse de dégradation correspond à un vitesse d'infiltration cellulaire.
